# EUROPEAN PATENT APPLICATION

(11) **EP 4 628 496 A1**
(43) Date of publication of application: **08.10.2025**
(21) Application number: 23910810.3
(22) Date of filing: 28.12.2023
(51) Int. Cl.: C07J 43/00, A61K 9/51, A61K 9/127, A61K 38/00, A61K 31/7088, A61K 31/713, A61K 35/76, A61P 31/14

(54) **STEROID-CATIONIC LIPID COMPOUND AND USE THEREOF**

(30) Priority: 29.12.2022 CN 202211708849
(71) Applicant: Jenkem Technology Co., Ltd. (Liaoning), Panjin, Liaoning 124000 (CN); Cansino (Shanghai) Biological Research Co., Ltd., Shanghai 201203 (CN)
(72) Inventor: YAN, Shengyong, Liaoning 124000 (CN); YAN, Zhihong, Shanghai 201203 (CN); HAO, Jing, Liaoning 124000 (CN); WANG, Haomeng, Shanghai 201203 (CN); WANG, Qingbin, Liaoning 124000 (CN); LIU, Jian, Shanghai 201203 (CN); MI, Jun, Liaoning 124000 (CN); ZHU, Tao, Shanghai 201203 (CN); GUO, Jun, Liaoning 124000 (CN); QIU, Dongxu, Shanghai 201203 (CN); ZHAO, Xuan, Liaoning 124000 (CN); YU, Xuefeng, Shanghai 201203 (CN)
(74) Representative: HGF
(86) International application number: PCT/CN2023/142644
(87) International publication number: WO 2024/140893

(57) **Abstract**

A steroid-cationic lipid compound as represented by formula (I). The LNP prepared from the compound can deliver a bioactive substance to a target cell or organ in an effective and stable manner, and the mRNA LNP prepared from the compound has good levels of stability and transfection efficiency, and can trigger a relatively high specific antibody response and cellular immune response in an animal.

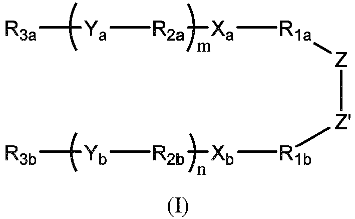

## Description

### TECHNICAL FIELD

The present invention belongs to the field of biopharmaceuticals, and particularly relates to a steroid-cationic lipid compound and use thereof in the delivery of a bioactive substance.

### BACKGROUND

Cationic liposome is a non-viral vector with a cell-like structure and biofilm properties, which can form a stable complex with RNA drugs having electronegativity by its electropositivity for delivering RNA drugs. Without precedence, *N*-(1-(2,3-diallyloxy)propyl)-*N,N,N*-trimethylammonium chloride (DOTMA) was used as a gene vector to carry DNA for cell transfection in 1987 by Felgner et al. Subsequently, extensive research on cationic liposomes as gene vectors in the field of gene therapy started to be performed, both domestically and abroad, by researchers.

However, the wide clinical use of cationic liposomes is subject to safety concerns and low transfection efficiency. Specifically, the cationic liposome-related products may have a positively charged surface, which is prone to nonspecific adsorption with negatively charged serum proteins, and this results in the formation of a large-size agglomerate that is easily eliminated by a mononuclear phagocyte system, leading to deteriorated circulation ability of the cationic liposome-gene complex in blood and reduced transfection efficiency. Therefore, the cationic liposomes need to be modified to obtain cationic liposomes that serve the purposes of safety, low-toxicity, and stability.

Many modified cationic lipids have been reported in the prior art. For example, patent document CN103930398A disclosed a lipid having the following structure: 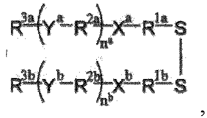 which is capable of achieving high intracellular expression efficiency. Patent document CN 114787127A disclosed a lipid having the general formula: 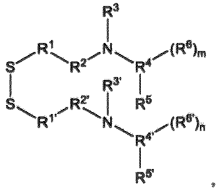 , and further provided a lipid nanoparticle (LNP) composition; patent document CN 114929205 A disclosed a lipid having the following general formula: 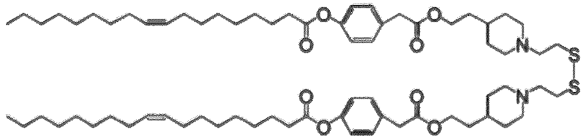 ; patent document WO 2021/089030A1 disclosed a compound having the general formula: 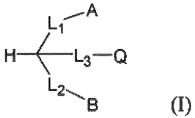 and use thereof for preparing a nucleic acid delivery agent. In the prior art, the modified cationic lipids are generally prepared into an LNP delivery system with ingredients of steroids, neutral lipids, PEG-lipids, nucleic acid drugs, etc. For example, patent document AU2020325221A1 disclosed a composition for target cell delivery of LNPs, comprising: (i) an ionizable lipid, (ii) a sterol or other structural lipids, (iii) a non-cationic helper lipid or a phospholipid, (iv) a PEG lipid, and (v) an agent (e.g., a nucleic acid molecule) encapsulated in and/or associated with the LNP, wherein the four components in a particular ratio enhance the delivery efficiency to the target cell. Patent document WO2021/250263 A1 disclosed a lipid composition comprising an ionizable lipid, a phospholipid, a sterol, a PEG lipid, and one or more nucleic acids, and disclosed a lipid composition comprising less than approximately 1 mol% of C14-PEG2000 lipid and specific percentages of other lipids. Patent CN102712935B disclosed a lipid particle, comprising: a cationic lipid; a neutral lipid, a zwitterionic lipid or an anionic lipid; a PEG-lipid; a sterol and a nucleic acid, wherein those components were assembled into lipid particles with a solid core, during which higher solid core-coating efficiency can be achieved. Patent document WO 2021/026358 Al disclosed a lipid nanoparticle (LNP) for target cell delivery, comprising: (i) an ionizable lipid, (ii) a sterol or other structural lipids, (iii) a non-cationic helper lipid or a phospholipid, (iv) a payload, and (v) a polyethylene glycol lipid, wherein the LNP, as a drug delivery system, serves the purposes of both safety and effectiveness.

As can be seen, in the prior art, by optimizing the amounts of components in the composition as well as the cationic lipid, a relatively safe, effective, and stable LNP delivery system is obtained. To further obtain a more effective, safer, and more stable LNP delivery system, the components and the structure of the components need to be further developed.

### SUMMARY

An objective of the present invention is to provide a cationic lipid compound, and a preparation method therefor and use thereof. The LNP prepared from the cationic lipid compound of the present invention has safety and efficacy, good biocompatibility, high *in vivo* mRNA transfection efficiency, high stability, and high biological safety.

To achieve the aforementioned objective, the present invention provides a cationic lipid compound, having a structure as shown in formula (I): 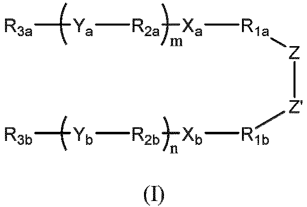 or a stereoisomer, a tautomer, and a pharmaceutically acceptable salt thereof, wherein,
Xₐ and X_{b} are each independently selected from 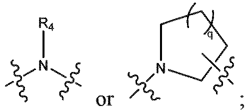
R₄ is C₁-C₆ alkyl; q is an integer of 1 or 2;
Z and Z' are each independently selected from S, C, or a chemical bond;
R₁ₐ and R_{1b} are each independently selected from C₁₋₆ alkylene or C₃-C₈ cycloalkylene;
R₂ₐ and R_{2b} are each independently selected from C₁₋₆ alkylene;
Yₐ and Y_{b} are independently selected from: -O-, -O(C=O)O-, -(C=O)NRa-, -NRa(C=O)-, -NRa-, -O(C=O)-, - (C=O)O-, -C(=O)-, -S(O)x-, -S-S-, -C(=O)S-, -SC(=O)-, -NRaC(=O)NRa-, -OC(=O)NRa-, -NRaC(=O)O-, - OC(=O)S-, or -SC(=O)O-;
Rₐ is H or C₁-C₁₂ alkyl, including but not limited to, C1, C2, C3, C4, C5, C6, C7, C8, C9, C10, C11, or C12 alkyl; m and n are each independently selected from **1, 2,** or 3;
R₃ₐ and R_{3b} are selected from a naturally occurring or non-naturally occurring steroid group, a fat-soluble vitamin residue, C₆-C₂₄ alkyl, C₆-C₂₄ monoalkenyl or polyalkenyl, or C₆-C₂₄ monoalkynyl or polyalkynyl, wherein CH₂ on the alkyl, alkenyl, or alkynyl is optionally replaced by one or more oxygen atoms;
the C₆-C₂₄ alkyl includes, but is not limited to C6, C7, C8, C9, C10, C11, C12, C13, C14, C15, C16, C17, C18, C19, C20, C22, C23, and C24 alkyl;
the C₆-C₂₄ monoalkenyl or polyalkenyl includes, but is not limited to, C6, C7, C8, C9, C10, C11, C12, C13, C14, C15, C16, C17, C18, C19, C20, C22, C23, and C24 monoalkenyl or polyalkenyl;
the C₆-C₂₄ monoalkynyl or polyalkynyl includes, but is not limited to C6, C7, C8, C9, C10, C11, C12, C13, C14, C15, C16, C17, C18, C19, C20, C22, C23, and C24 monoalkynyl or polyalkynyl;
provided that at least one of R₃ₐ and R_{3b} is a naturally occurring or non-naturally occurring steroid group.

In some embodiments, the cationic lipid compound as shown in formula (I) of the present invention is a cationic lipid compound as shown in formula (II), 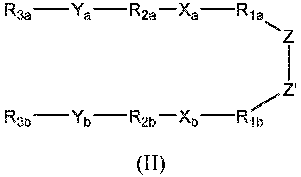 or a stereoisomer, a tautomer, and a pharmaceutically acceptable salt thereof, wherein,
Xₐ and X_{b} are each independently selected from 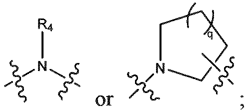
R₄ is C₁-C₆ alkyl; q is an integer of 1 or 2;
Z and Z' are each independently selected from S, C, or a chemical bond;
R₁ₐ and R_{1b} are each independently selected from C₁₋₆ alkylene or C₃-C₈ cycloalkylene;
R₂ₐ and R_{2b} are each independently selected from C₁₋₆ alkylene;
Yₐ and Y_{b} are independently selected from: -O-, -O(C=O)O-, -(C=O)NRa-, -NRa(C=O)-, -NRa-, -O(C=O)-, - (C=O)O-, -C(=O)-, -S(O)x-, -S-S-, -C(=O)S-, -SC(=O)-, -NRaC(=O)NRa-, -OC(=O)NRa-, -NRaC(=O)O-, - OC(=O)S-, or -SC(=O)O-;
Rₐ is H or C₁-C₁₂ alkyl;
R₃ₐ and R_{3b} are selected from a naturally occurring or non-naturally occurring steroid group, a fat-soluble vitamin residue, C₆-C₂₄ alkyl, C₆-C₂₄ monoalkenyl or polyalkenyl, or C₆-C₂₄ monoalkynyl or polyalkynyl, wherein CH₂ on the alkyl, alkenyl, or alkynyl is optionally replaced by one or more oxygen atoms;
provided that at least one of R₃ₐ and R_{3b} is a naturally occurring or non-naturally occurring steroid group.

In some embodiments, in the cationic lipid compound of formula (I) and/or formula (II) of the present invention, Xₐ and X_{b} are selected from 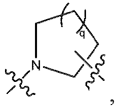 wherein q is an integer of 1 or 2.

In some embodiments, in the cationic lipid compound as shown in formula (I), Z and Z' are each independently selected from S or C.

In some embodiments, in the cationic lipid compound as shown in formula (I), Z and Z' are each independently selected from S.

In some embodiments, the cationic lipid compound of formula (I) and/or formula (II) of the present invention is a compound as shown in formula (III-1), formula (III-2), or formula (III-3), 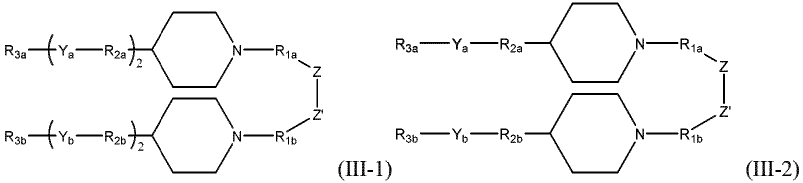 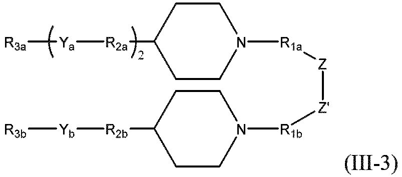 or a stereoisomer, a tautomer, and a pharmaceutically acceptable salt thereof, wherein Z, Z', Yₐ, Y_{b}, R₁ₐ, R_{1b}, R₂ₐ, R_{2b}, R₃ₐ, and R_{3b} are as defined herein.

In some embodiments, in the cationic lipid compound of the present invention,
R₁ₐ and R_{1b} are each independently C₁-C₆ alkylene, including but not limited to, C1, C2, C3, C4, C5, and C6 alkyl; R₂ₐ and R_{2d} are each independently C₁-C₃ alkylene, including but not limited to, C1, C2, and C3 alkyl;
Yₐ and Y_{b} are each independently selected from: -O-, -O(C=O)O-, -O(C=O)-, -(C=O)O-, or -C(=O)-;
R₃ₐ and R_{3b} are selected from a naturally occurring or non-naturally occurring steroid group, a fat-soluble vitamin residue, C₆-C₂₄ alkyl, C₆-C₂₄ monoalkenyl or polyalkenyl, or C₆-C₂₄ monoalkynyl or polyalkynyl, wherein CH₂ on the alkyl, alkenyl, or alkynyl is optionally replaced by one or more oxygen atoms; at least one of R₃ₐ and R_{3b} is a naturally occurring or non-naturally occurring steroid group.

In some embodiments, in the cationic lipid compound of the present invention, the steroid is selected from avenasterol, β-sitosterol, brassicasterol, ergocalciferol, campesterol, cholestanol, cholesterol, coprostanol, dehydrocholesterol, desmosterol, dihydroergocalciferol, dihydrocholesterol, dihydroergosterol, dinosterol, epicholesterol, ergosterol, fucosterol, hexahydrolumisterol, hydroxycholesterol, lanosterol, lumisterol, saringosterol, sitostanol, sitosterol, stigmastanol, stigmasterol, cholic acid, glycocholic acid, taurocholic acid, deoxycholic acid, and/or lithocholic acid.

In some embodiments, the steroid group as described herein is a sterol group.

In some embodiments, the sterol as described herein is a zoosterol, or an oxidized or reduced form thereof; and/or, the sterol is a phytosterol, or an oxidized or reduced form thereof; and/or, the sterol is a synthetic sterol, or an oxidized or reduced form thereof.

In some embodiments, the sterol as described herein is selected from cholesterol, an oxidized form of cholesterol, a reduced form of cholesterol, an alkyl ester of lithocholic acid, stigmasterol, stigmastanol, campesterol, ergosterol, and/or sitosterol.

In some embodiments, the sterol as described herein is an oxidized form of cholesterol, a reduced form of cholesterol, an alkyl ester of lithocholic acid, stigmasterol, stigmastanol, campesterol, ergosterol, and sitosterol.

In some embodiments, the steroid group as described herein has the structure as shown below: 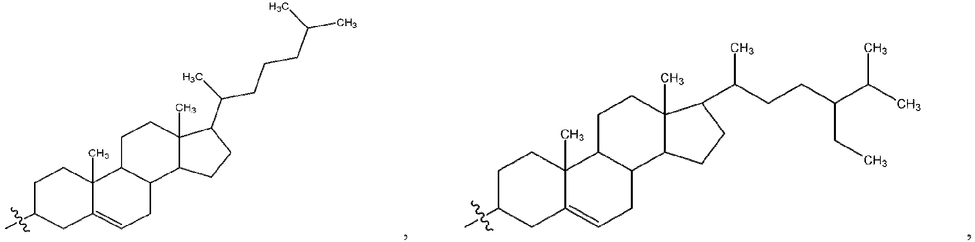 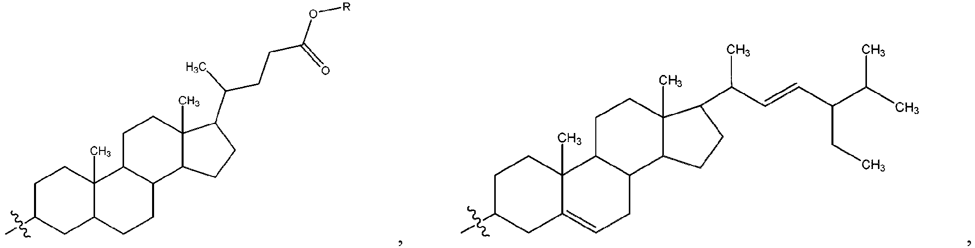 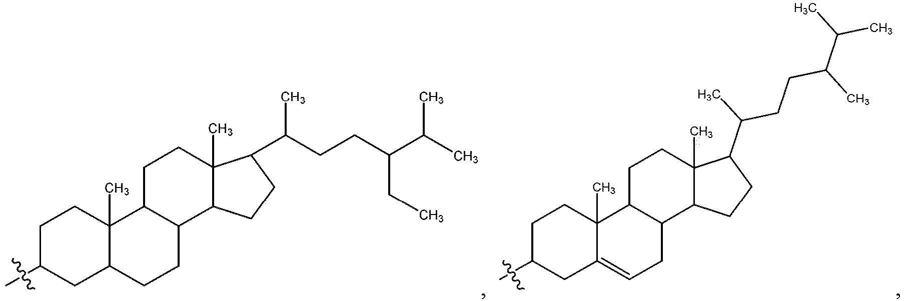 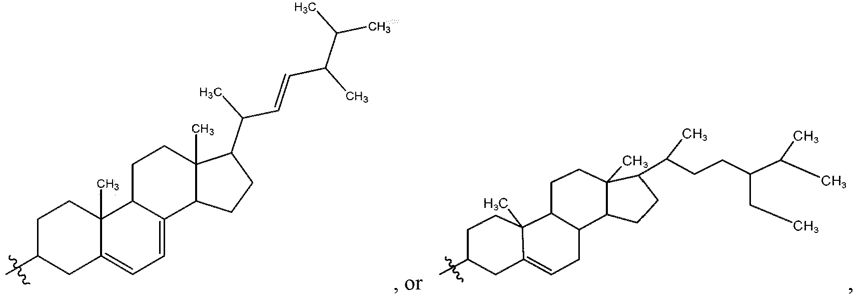 wherein R is C₁₋₂₀ alkyl, including but not limited to, C1, C2, C3, C4, C5, C6, C7, C8, C9, C10, C11, C12, C13, C14, C15, C16, C17, C18, C19, and C20 alkyl.

In some embodiments, the cationic lipid compound of formula (I) and/or formula (II) of the present invention is a compound as shown in formula (IV-1), formula (IV-2), formula (IV-3), formula (IV-4), formula (IV-5), formula (IV-6), or formula (IV-7), 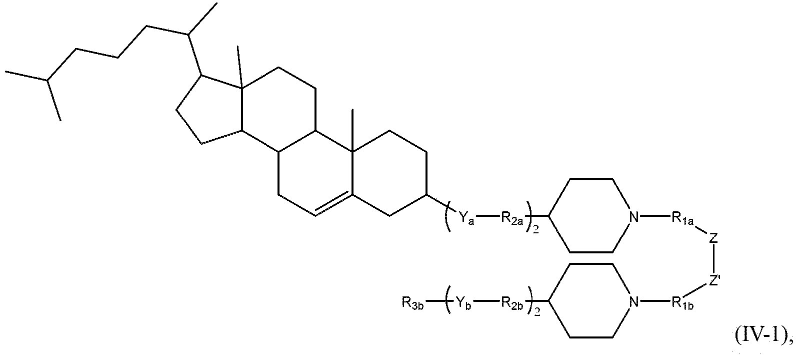 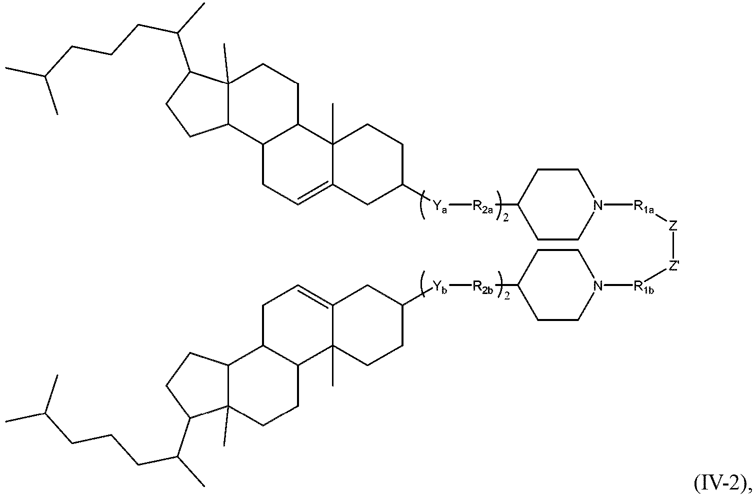 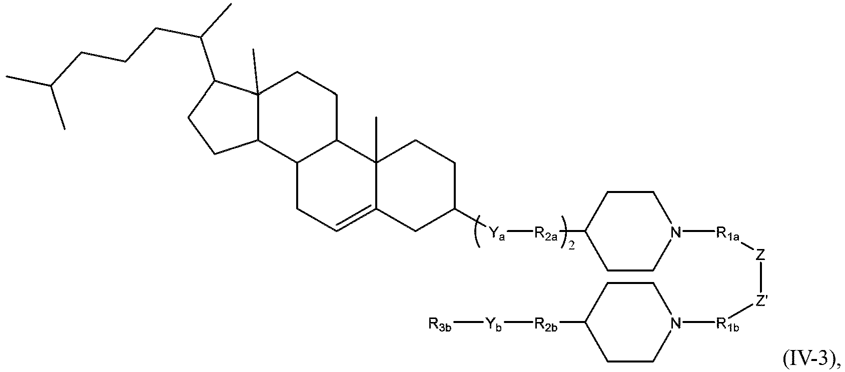 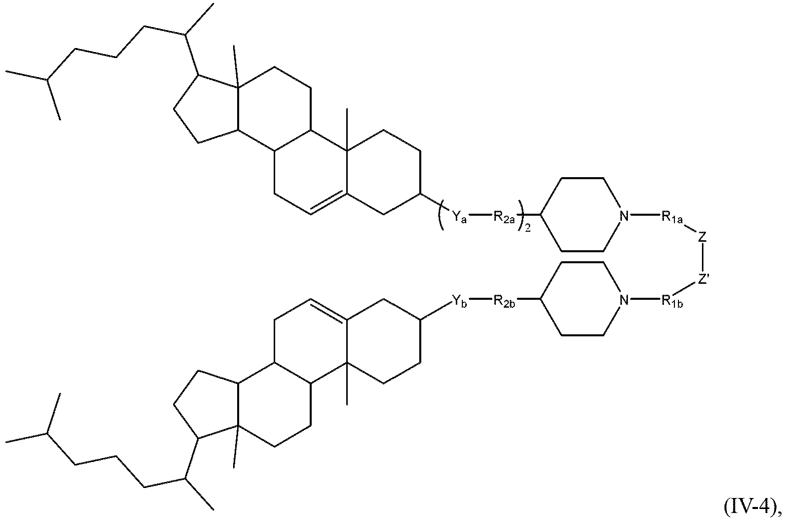 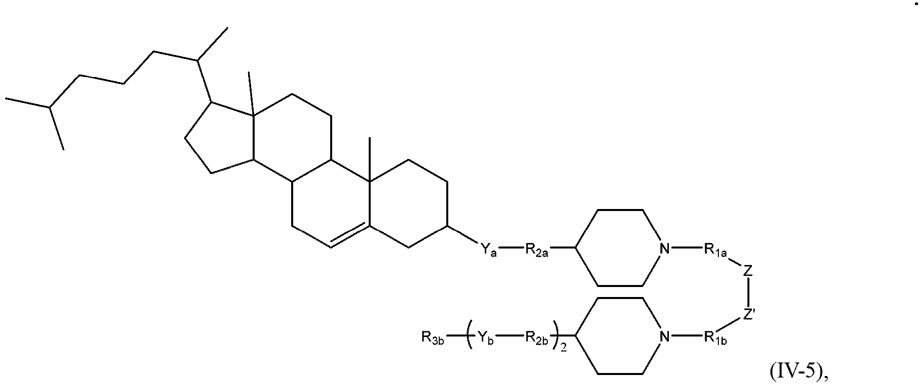 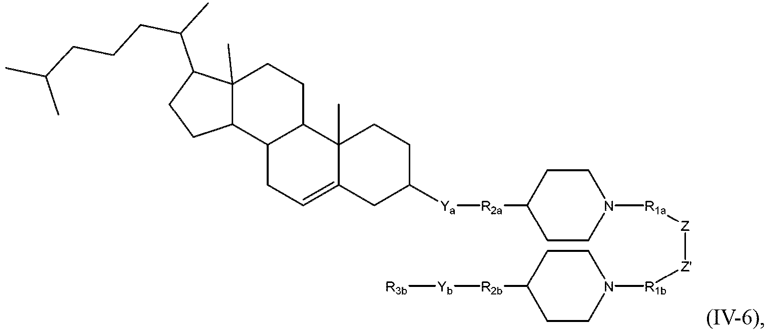 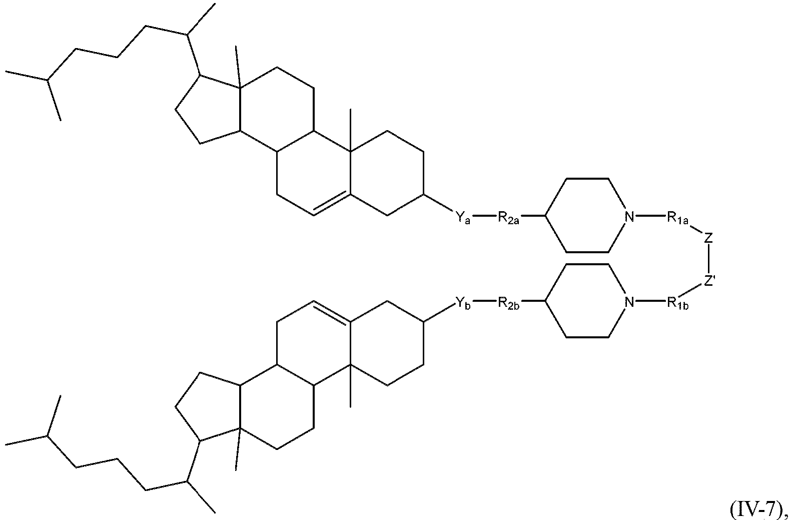 or a stereoisomer, a tautomer, and a pharmaceutically acceptable salt thereof, wherein R_{3b} is selected from a fat-soluble vitamin residue, C₆-C₂₄ alkyl, C₆-C₂₄ monoalkenyl or polyalkenyl, C₆-C₂₄ monoalkynyl or polyalkynyl, wherein CH₂ on the alkyl, alkenyl, or alkynyl is optionally replaced by one or more oxygen atoms; Z, Z', Yₐ, Y_{b}, R₁ₐ, R_{1b}, R₂ₐ, and R_{2b} are as defined herein.

In some embodiments, the cationic lipid compound of formula (I) and/or formula (II) of the present invention is a compound as shown in formula (V-1), formula (V-2), formula (V-3), formula (V-4), formula (V-5), formula (V-6), or formula (V-7), 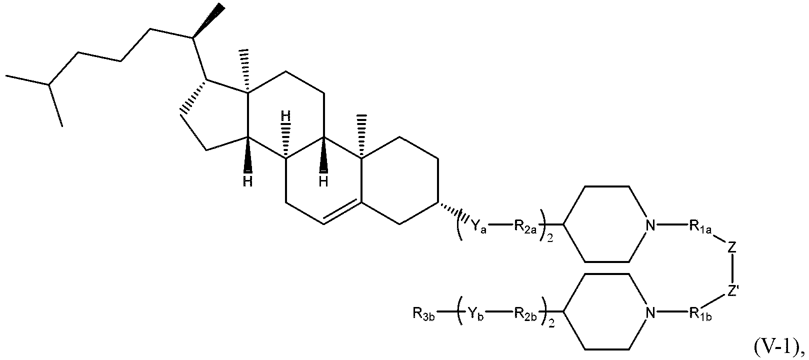 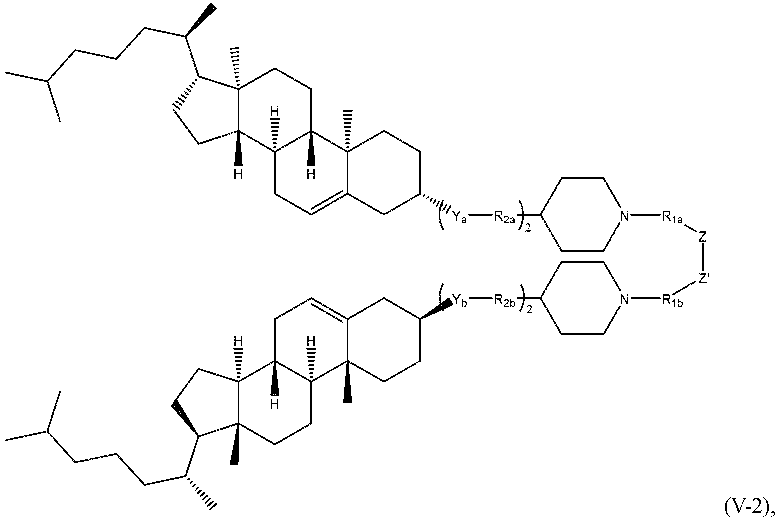 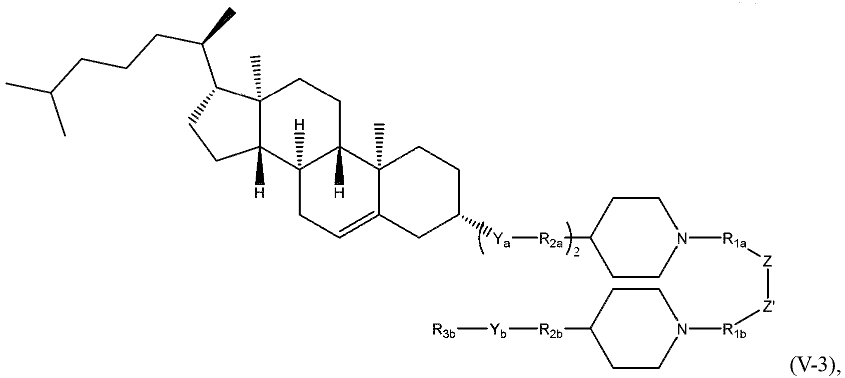 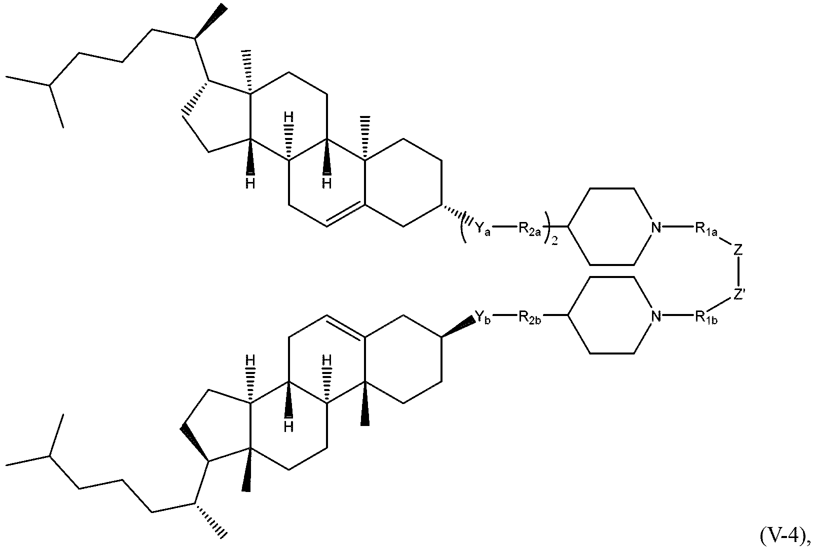 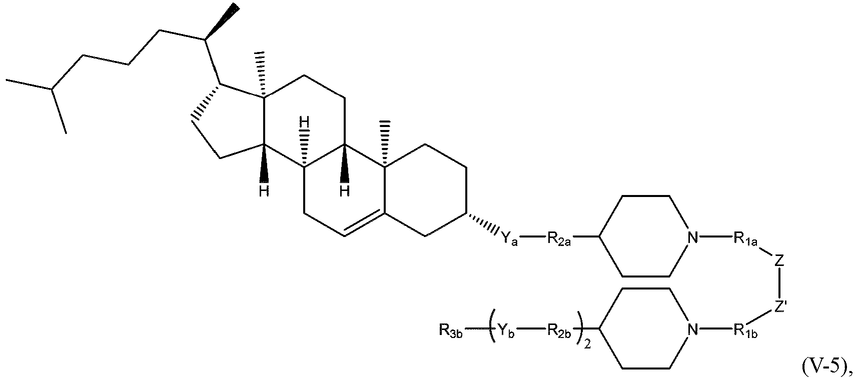 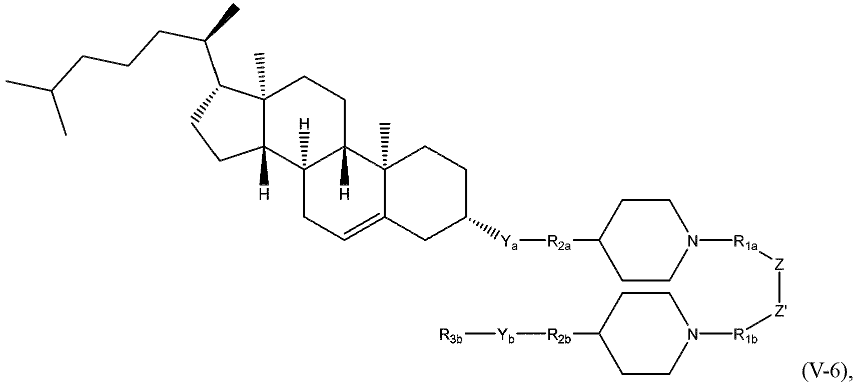 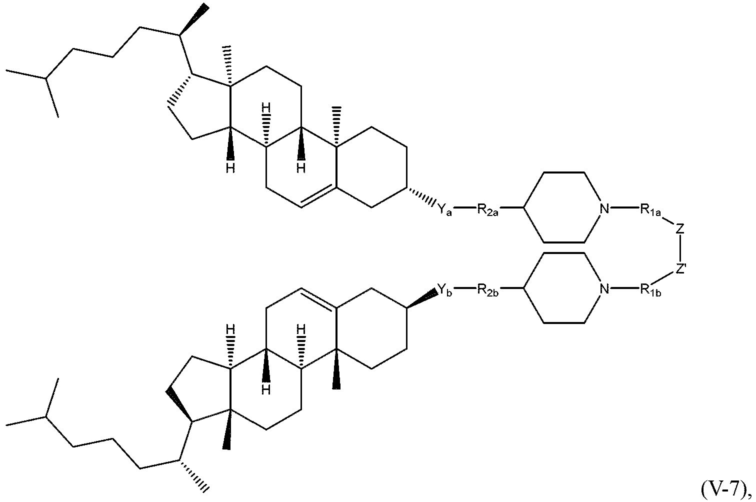 or a stereoisomer, a tautomer, and a pharmaceutically acceptable salt thereof, wherein R_{3b} is selected from a fat-soluble vitamin residue, C₆-C₂₄ alkyl, C₆-C₂₄ monoalkenyl or polyalkenyl, C₆-C₂₄ monoalkynyl or polyalkynyl, wherein CH₂ on the alkyl, alkenyl, or alkynyl is optionally replaced by one or more oxygen atoms; Z, Z', Yₐ, Y_{b}, R₁ₐ, R_{1b}, R₂ₐ, and R_{2b} are as defined herein.

In some embodiments, in the cationic lipid compound of the present invention, R_{3b} is selected from: ,

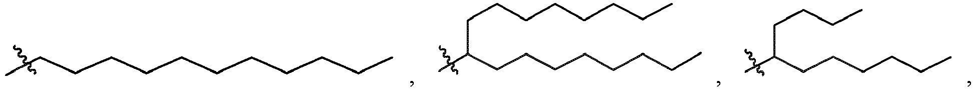

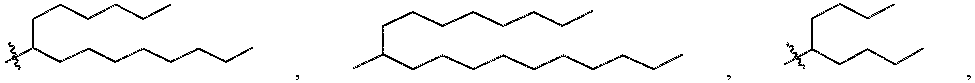

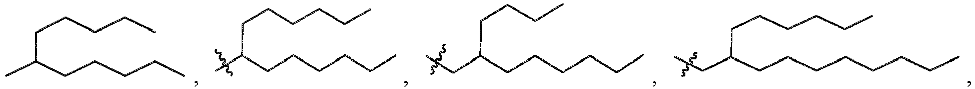

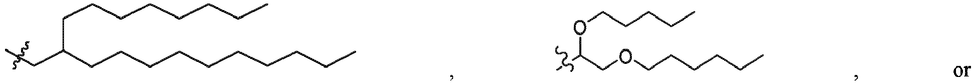

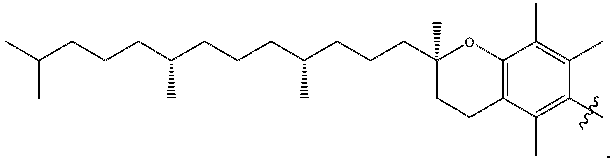

In some embodiments, in the cationic lipid compound of the present invention, the C₆-C₂₄ alkyl comprises any one of the following: ,

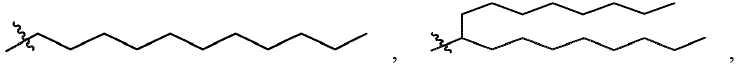

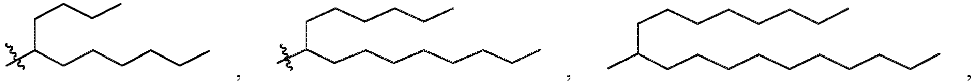

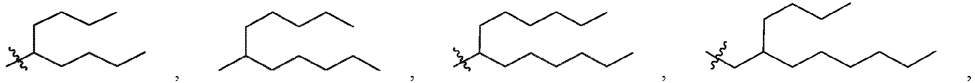

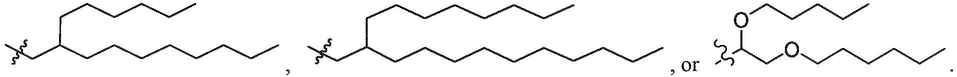

In some embodiments, the cationic lipid compound of the present invention is selected from: 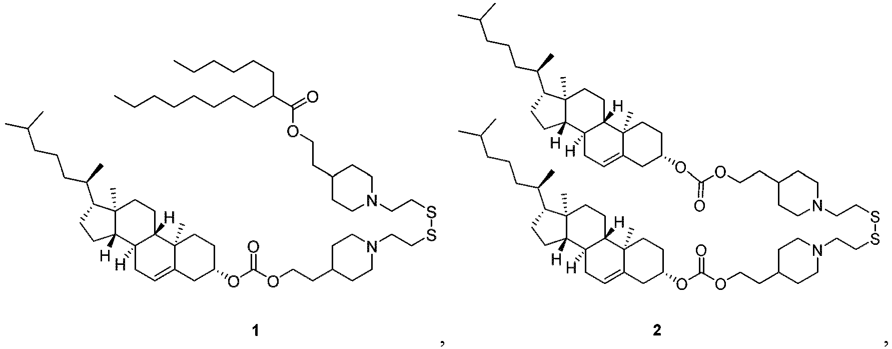 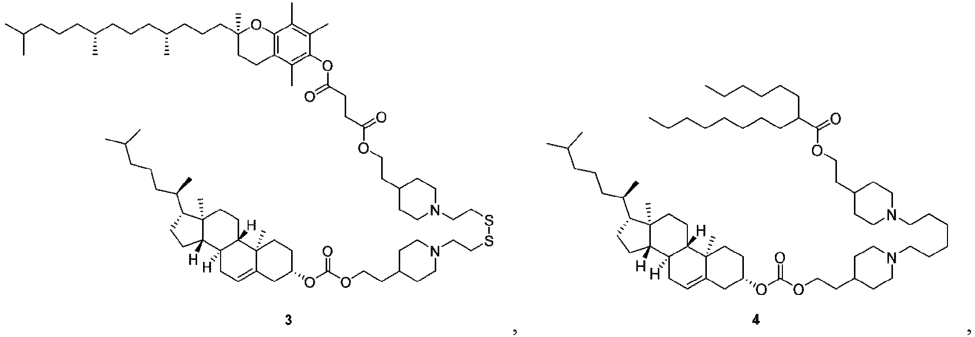 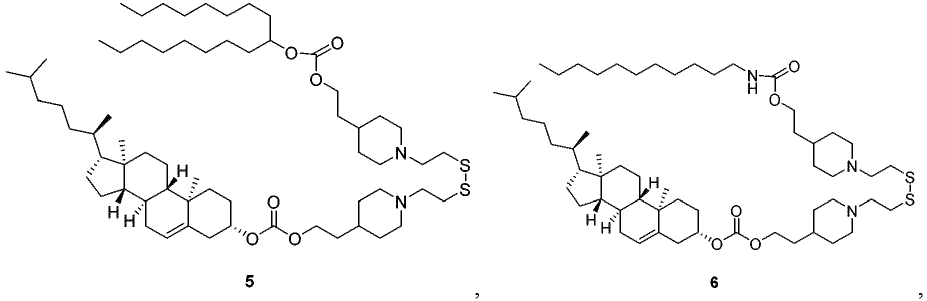 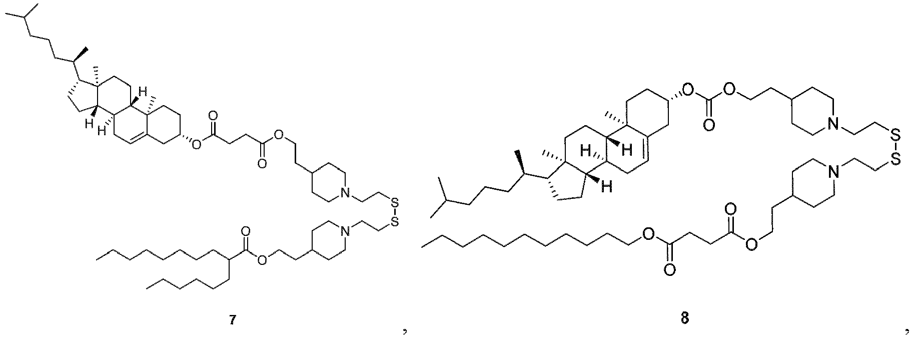 or 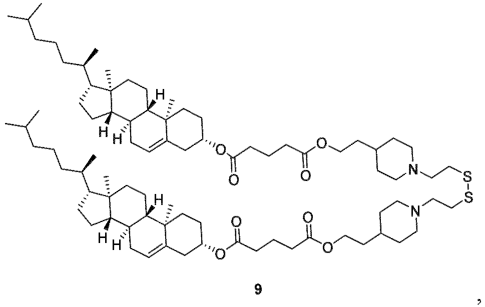 or a stereoisomer, a tautomer, and a pharmaceutically acceptable salt thereof.

In some embodiments, the present invention provides an LNP, comprising the cationic lipid compound as described in the present invention.

In some embodiments, the LNP as described in the present invention further comprises a polyethylene glycol lipid and at least one second lipid, wherein the second lipid is selected from: a neutral lipid, a zwitterionic lipid, or an anionic lipid.

In some embodiments, the polyethylene glycol lipid is selected from: 2-[(polyethylene *glycol)-2000]-N,N-*ditetradecylacetamide (ALC-0159), 1,2-dimyristoyl-sn-glycerol methoxypolyethylene glycol (PEG-DMG), 1,2-distearoyl-sn-glycero-3-phosphoethanolamine-N-[amino(polyethylene glycol)] (PEG-DSPE), PEG-disterol glycerol (PEG-DSG), PEG-dipalmitoyl, PEG-dioleyl, PEG-distearyl, PEG-diacylglycerol amide (PEG-DAG), PEG-dipalmitoyl phosphatidylethanolamine (PEG-DPPE), or PEG-1,2-dimyristoyloxypropyl-3-amine (PEG-c-DMA);
and/or,
the neutral lipid is selected from: 1,2-distearoyl-sn-glycero-3-phosphocholine (DSPC), 1,2-dipalmitoyl-sn-glycero-3-phosphocholine (DPPC), 1,2-dioleoyl-sn-glycero-3-phosphoethanolamine (DOPE), 1,2-dipalmitoyl-sn-glycero-3-phosphoethanolamine (DPPE), 1,2-dimyristoyl-sn-glycero-3-phosphoethanolamine (DMPE), 2-dioleoyl-sn-glycero-3-phospho-(1'-rac-glycerol) (DOPG), oleoyl phosphatidylcholine (POPC), or 1-palmitoyl-2-oleoyl phosphatidylethanolamine (POPE).

In some embodiments, in the LNP, the molar ratio of the cationic lipid as described in the present invention to the second lipid to the PEG-lipid is in the range of 10:20:1 to 60:80:30; preferably, the molar ratio of the cationic lipid as described in the present invention to the second lipid to the PEG-lipid is in the range of 40:40:1 to 60:60:10; more preferably, the molar ratio of the cationic lipid as described in the present invention to the second lipid to the PEG-lipid is 49:49:2 and/or 49.25:49.25:1.5.

In some embodiments, the nitrogen-to-phosphorus ratio of the LNP is in the range of 1:1 to 15:1, for example, the nitrogen-to-phosphorus ratio may be 1:1, 1.5:1, 2:1, 2.5:1, 3:1, 3.5:1, 4:1, 4.5:1, 5:1, 5.5:1, 6:1, 6.5:1, 7:1, 7.5:1, 8:1, 8.5:1, 9:1, 9.5:1, 10:1, 10.5:1, 11:1, 11.5:1, 12:1, 12.5:1, 13:1, 13.5:1, 14:1, 14.5:1, or 15:1; preferably, the nitrogen-to-phosphorus ratio of the LNP is in the range of 2:1 to 10:1; more preferably, the nitrogen-to-phosphorus ratio of the LNP is in the range of 4:1 to 8:1; most preferably, the nitrogen-to-phosphorus ratio of the LNP is 8:1. In some embodiments, the diameter of the LNP is 15-300 nm, including but not limited to, 15 nm, 20 nm, 25 nm, 30 nm, 35 nm, 40 nm, 45 nm, 50 nm, 55 nm, 60 nm, 65 nm, 70 nm, 80 nm, 100 nm, 150 nm, 200 nm, 250 nm, or 300 nm. Preferably, the diameter of the LNP is 50-100 nm.

In the present invention, physical and chemical factors such as particle size, electrical charge, and surface properties may affect LNP delivery, and a suitable LNP particle size can allow a bioactive substance to efficiently enter a targeted organ, enhancing the potency of LNP.

In some embodiments, the present invention further provides use of the cationic lipid compound as described in the present invention and an LNP prepared therefrom in the preparation of a bioactive substance delivery system.

In some embodiments, the bioactive substance as described herein may be a small molecule compound, a nucleic acid, an oligopeptide, etc.; preferably, the bioactive substance is a nucleic acid.

In some embodiments, the bioactive substance as described herein is a DNA or an RNA.

In some embodiments, the DNA as described herein comprises a non-coding DNA (an antisense DNA) or a coding DNA.

In some embodiments, the RNA as described herein comprises an antisense RNA, an saRNA, an mRNA, an lncRNA, an miRNA, an siRNA, a piRNA, a gRNA, a tsRNA, a circRNA, and a self-replicating mRNA.

In some embodiments, the nucleic acid is used for preventing and/or treating cancer, inflammation, fibrotic disease, autoimmune disease, infection, psychiatric disorder, hematological disorder, chromosomal disease, genetic disease, connective tissue disease, digestive disease, ear-nose-throat disease, endocrine disease, eye disease, reproductive disease, heart disease, kidney disease, lung disease, metabolic disorder, oral disease, musculoskeletal disease, neonatal screening, nutritional disease, parasitic disease, skin disease, etc.

In some embodiments, the bioactive substance delivery system is an mRNA vaccine.

In some embodiments, the mRNA vaccine may be used for preventing cancer, viral infection, bacterial infection, fungal infection, etc. The virus comprises, but is not limited to: norovirus, Ebola virus, coronavirus (including novel coronavirus SARS-CoV-2), cytomegalovirus, Dengue virus, Zika virus, coxsackievirus, enterovirus, hepatitis virus, herpes simplex virus, human papilloma virus, influenza virus, Marburg virus, measles virus, poliovirus, rabies virus, rotavirus, measles virus, etc.

In some embodiments, the present invention further provides use of the LNP for preparing an mRNA vaccine. The preparation method of the LNP of the present invention may be a method conventional in the art, e.g., a microfluidic technique.

In some embodiments, the present invention further provides a medicament, comprising the bioactive substance and the LNP as described herein.

In some embodiments, the medicament further comprises a pharmaceutically acceptable auxiliary material.

In some embodiments, the pharmaceutically acceptable auxiliary material as described in the present invention is, e.g., a carrier, an adjuvant, a diluent, etc.

In some embodiments, the steroid-cationic lipid compound, the LNP, or the medicament as described in the present invention may deliver the bioactive substance by means of oral administration, inhalation, or injection.

In some embodiments, the medicament as described in the present invention is a gene drug.

In some embodiments, the present invention further provides a method for delivering a bioactive substance, which comprises administering to a human population in need thereof the medicament as described in the present invention.

The present invention has the following beneficial effects:
(1) The cationic lipid compound with a specific structure as described in the present invention has a controllable particle size, a uniform distribution, monodispersity, and high encapsulation efficiency for negatively charged drugs, and serves the purposes of low biotoxicity and good stability.
(2) The cationic lipid compound as described in the present invention has relatively good stability and transfection efficiency as an mRNA LNP, and can cause relatively high specific antibody response and cellular immune response in a laboratory animal.

### BRIEF DESCRIPTION OF THE DRAWINGS

FIG. 1 shows the transfection efficiency of Luc-mRNA-LNPs in HEK293T cells by a bioluminescence assay.
FIG. 2a shows serum antigen-specific antibody titers in the mice after being immunized with novel coronavirus antigen mRNA-LNPs by an ELISA assay.
FIG. 2b shows IFN-γ+ cell response levels in PBMCs in the mice after being immunized with novel coronavirus antigen mRNA-LNPs by an ELSPOT assay.
FIG. 2c shows specific CD4+ T cell response levels in the spleens of the mice after being immunized with novel coronavirus antigen mRNA-LNPs by an ICS assay.
FIG. 2d shows specific CD8+ T cell response levels in the spleens of the mice after being immunized with novel coronavirus antigen mRNA-LNPs by an ICS assay.

### DETAILED DESCRIPTION

The technical solutions in the examples of the present invention will be described clearly and completely below with reference to the drawings. It is obvious that the described examples are only a part of the examples of the present invention, rather than all of them. Based on the examples of the present invention, all other examples obtained by those of ordinary skills in the art without creative work shall fall within the protection scope of the present invention.

The term "LNP (Lipid Nanoparticle)" of the present invention refers to a particle having a nanometer scale, e.g., a particle of 1 nm to 1,000 nm, comprising one or more types of Lipid molecules.

The term "gene drug" of the present invention generally consists of a vector or a delivery system containing an engineered gene construct, the active ingredient of which may be a DNA, an RNA, or a genetically modified virus, bacterium, or cell. By introducing an exogenous gene into a target cell or tissue to replace, compensate for, inhibit, and modify a specific gene, the purpose of treating and preventing a disease can be achieved.

The term "nucleic acid" of the present invention refers to a polymer containing at least two deoxyribonucleotides or ribonucleotides in either single- or double-stranded form and includes DNA, RNA, and hybrids thereof.

The term "lipid" of the present invention refers to a group of organic compounds that include, but are not limited to, esters of fatty acids and are generally characterized by being poorly soluble in water, but soluble in many organic solvents.

The term "cationic lipid" of the present invention refers to a lipid molecule capable of being positively charged. The term "neutral lipid" of the present invention refers to an uncharged non-phosphoglyceride lipid molecule.

The term "polyethylene glycol lipid" of the present invention refers to a molecule comprising a lipid portion and a polyethylene glycol portion.

The term "delivery system" of the present invention refers to a formulation or composition that regulates the spatial, temporal, and dose distribution of a bioactive ingredient in an organism.

### Example 1: Synthesis of Compound 1

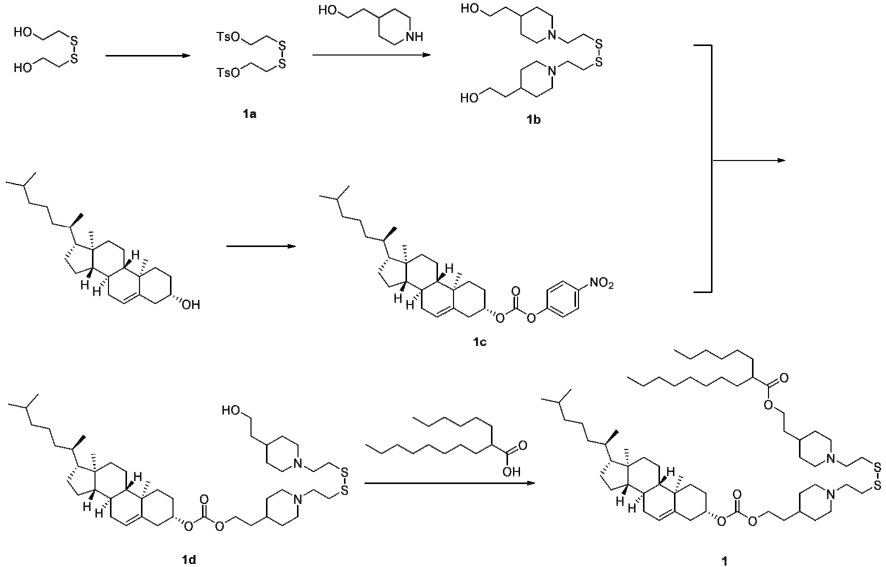

### Step 1: Synthesis of compound 1a

Bis(2-hydroxyethyl)disulfide (100.0 g, 0.648 mol) was dissolved in 2.0 L of dichloromethane, and triethylamine (196.8 g, 1.945 mol) was added. The resulting mixture was cooled to 0 °C on an ice water bath, and *p-*toluenesulfonyl chloride (296.5 g, 1.555 mol) was added. After the completion of material addition, the mixture was heated to room temperature and stirred for 16 h. Samples were taken for an HPLC assay to confirm the completion of the reaction. The reaction solution was washed twice sequentially with a saturated aqueous sodium bicarbonate solution and water. The organic phase was dried over anhydrous sodium sulfate and concentrated to give a brown oil. Ethyl acetate was added followed by slow addition of n-hexane with stirring, and the stirring was kept for 30 min. The reaction solution was filtered to give a filter residue, which dried under vacuum at room temperature for 16 h to give compound 1a (255 g, white solid) in a yield of 85%.

### Step 2: Synthesis of compound 1b

Compound 1a (255.0 g, 0.551 mol) was dissolved in 3.8 L of acetonitrile, and potassium carbonate (228.2 g, 1.654 mol) and 2-(piperidin-4-yl)ethanol (213.7 g, 1.654 mol) were added. The resulting mixture was heated to 40 °C and stirred for 16 h, and the reaction was completed as monitored by HPLC. The reaction solution was filtered through celite. The filter cake was washed with dichloromethane and the filtrate was concentrated until no liquid evaporated off. Dichloromethane was added, and the resulting mixture was washed twice with 2.5 L of 5% aqueous sodium bicarbonate solution and subsequently washed twice with water. The organic phase was dried over anhydrous sodium sulfate and concentrated at 40 °C to give compound 1b (154 g, brown oil) in a yield of 74%.

### Step 3: Synthesis of compound 1c

Cholesterol (10.0 g, 25.86 mmol) was dissolved in 100 mL of dichloromethane, and DMAP (0.6 g, 5.17 mmol) and triethylamine (5.2 g, 51.71 mmol) were added. Subsequently, phenylp-nitrochloroformate (5.7 g, 28.44 mmol) was added in portions. The resulting mixture was stirred at room temperature for 16 h to allow the reaction, and the reaction was completed as monitored by TCL. The reaction solution was dropwise added to acetonitrile, and a solid precipitated. The solid was crystallized and filtered under vacuum, and the filter cake was dried to give compound 1c (13.0 g, white solid) in a yield of 91%.

### Step 4: Synthesis of compound 1d

Compound 1c (10.0 g, 18.12 mmol) was dissolved in 100 mL of dichloromethane, and compound 1b (6.8 g, 18.12 mmol), EDC·HCL (2.6 g, 13.59 mmol), and DMAP (0.22 g,1.81 mmol) were added. The resulting mixture was stirred at room temperature for 16 h, and the reaction was completed as monitored by TCL. The reaction solution was concentrated, dissolved by the addition of n-hexane, and washed twice with acetonitrile under stirring. The *n-*hexane phase was concentrated and separated by column chromatography to give compound 1d (9.7 g, light yellow oil) in a yield of 68%.

### Step 5: Synthesis of compound 1

Compound 1d (5.0 g, 6.30 mmol) was dissolved in 50 mL of dichloromethane, and 2-hexyldecanoic acid (1.63 g, 6.30 mmol), EDC·HCL (1.8 g, 9.41 mmol), and DMAP (0.16 g, 1.26 mmol) were added. The resulting mixture was stirred at room temperature for 16-20 h, and the reaction was completed as detected by CAD. The reaction solution was concentrated. Subsequently, the concentrated reaction solution was dissolved by the addition of n-hexane, and washed twice with acetonitrile. The n-hexane phase was concentrated to dryness and separated by column chromatography to give compound 1 (2.9 g, light yellow oil) in a yield of 45%.

MS m/z (ESI): 1027.9 [M+1].

1H NMR (300MHz, CDCl3): δ 5.45 (t, 1H, J = 5.4 Hz), 4.53-4.40 (m, 1H), 4.20-3.95 (m, 4H), 2.95-2.80 (m, 8H), 2.71-2.50 (m, 4H), 2.47-2.36 (m, 3H), 2.11-1.78 (m, 9H), 1.76-0.85 (m, 77H), 0.70 (s, 3H).

### Example 2: Synthesis of Compound 2

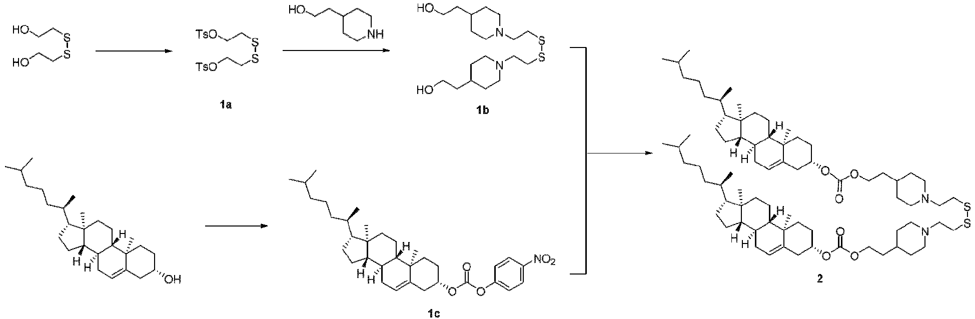

Compound 1c (10.0 g, 18.12 mmol) was dissolved in 100 mL of dichloromethane, and compound 1b (3.4 g, 9.06 mmol), EDC·HCL (5.19 g, 27.18 mmol), and DMAP (0.44 g, 3.62 mmol) were added. The resulting mixture was stirred at room temperature for 16 h, and the reaction was completed as monitored by TCL. The reaction solution was concentrated, dissolved by the addition of n-hexane, and washed twice with acetonitrile under stirring. The *n-*hexane phase was concentrated to dryness and separated by column chromatography to give compound 2 (4.0 g, brown solid) in a yield of 37%.

MS m/z (ESI): 1203.1 [M+1].

1H NMR (300MHz, CDCl3): δ 5.45 (t, 2H, J = 5.4 Hz), 4.53-4.40 (m, 2H), 4.20-3.95 (m, 4H), 2.95-2.80 (m, 8H), 2.71-2.50 (m, 4H), 2.47-2.36 (m, 4H), 2.11-1.78 (m, 14H), 1.76-0.85 (m, 80H), 0.70 (s, 6H).

### Example 3: Synthesis of Compound 3

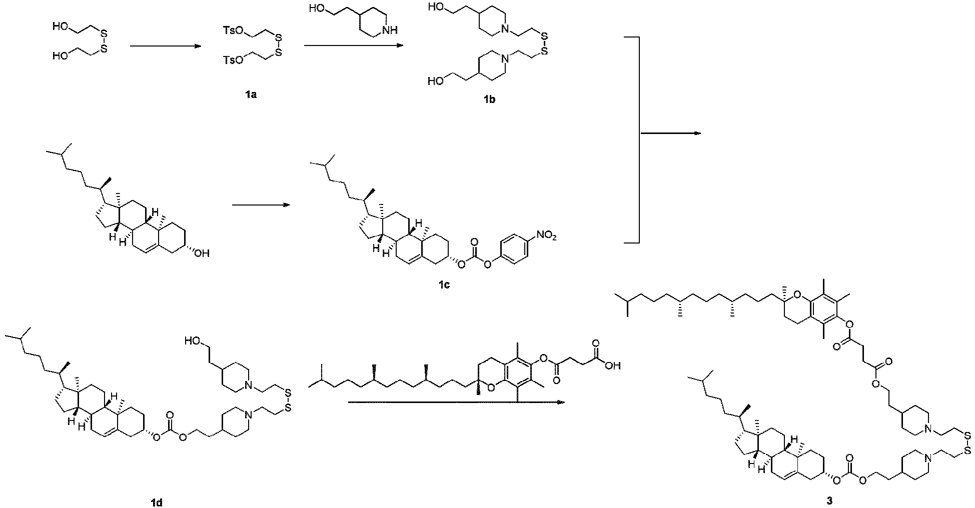

Compound 1d (5.0 g, 6.30 mmol) was dissolved in 50 mL of dichloromethane, and vitamin E succinate monoester (3.34 g, 6.30 mmol), EDC·HCL (1.8 g, 9.41 mmol), and DMAP (0.16 g, 1.26 mmol) were added. The resulting mixture was stirred at room temperature for 16-20 h, and the reaction was completed as detected by CAD. The reaction solution was concentrated, dissolved by the addition of n-hexane, and washed twice with acetonitrile. The *n*-hexane phase was concentrated to dryness and separated by column chromatography to give compound 3 (2.6 g, light yellow oil) in a yield of 32%.

MS m/z (ESI): 1303.2 [M+1].

1H NMR (300MHz, CDCl3): δ 5.45 (t, 1H, J = 5.4 Hz), 4.53-4.40 (m, 1H), 4.20-3.95 (m, 4H), 2.95-2.80 (m, 4H), 2.71-2.50 (m, 8H), 2.47-2.36 (m, 14H), 2.11-1.78 (m, 15H), 1.76-0.85 (m, 82H), 0.70 (s, 3H).

### Example 4: Synthesis of Compound 4

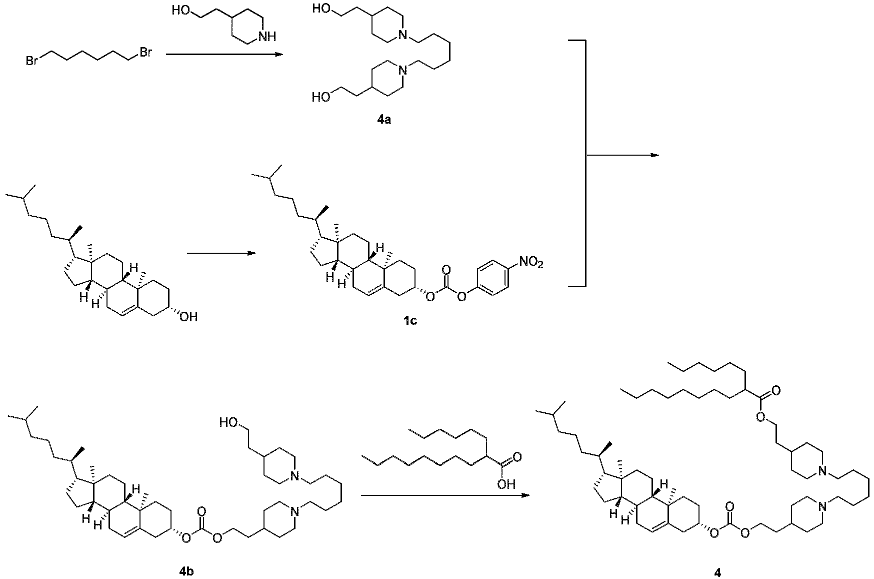

### Step 1: Synthesis of compound 4a

1,6-Dibromohexane (244 mg, 1.0 mmol) was dissolved in tetrahydrofuran, and acetonitrile, 2-(piperidin-4-yl)ethanol (223 mg, 2.5 mmol), potassium carbonate (550 mg, 4.0 mmol), and potassium iodide (332 mg, 2.0 mmol) were added, and the resulting mixture was stirred at 83 °C for 16-20 h. The reaction system was cooled to room temperature and filtered. The filter residue was washed with dichloromethane, and a saturated aqueous sodium bicarbonate solution was added to the obtained filtrate. The mixture was extracted twice with dichloromethane. The organic phases were combined, dried over anhydrous sodium sulfate, filtered and concentrated, and separated by column chromatography to give compound 4a (215 mg, light yellow oil) in a yield of 63%.

### Step 2: Synthesis of compound 4b

Compound 1c (552 mg, 1.0 mmol) was dissolved in 10 mL of dichloromethane, and compound 4a (341 mg, 1.0 mmol), EDC·HCL (288 mg, 1.5 mmol), and DMAP (25 mg, 0.2 mmol) were added. The resulting mixture was stirred at room temperature for 16 h, and the reaction was completed as monitored by TCL. The reaction solution was concentrated, dissolved by the addition of n-hexane, and subsequently washed twice with acetonitrile. The *n-*hexane phase was concentrated to dryness and separated by column chromatography to give compound 4b (535 mg, light yellow oil) in a yield of 71%.

### Step 3: Synthesis of compound 4

Compound 4b (753 mg, 1.0 mmol) was dissolved in 5 mL of dichloromethane, and 2-hexyldecanoic acid (253 mg, 1.0 mmol), EDC·HCL (288 mg, 1.5 mmol), and DMAP (25 mg, 0.2 mmol) were added. The resulting mixture was stirred at room temperature for 16-20 h, and the reaction was completed as detected by CAD. The reaction solution was concentrated to dryness, dissolved by the addition of n-hexane, and washed twice with acetonitrile. The *n-*hexane phase was concentrated to dryness and separated by column chromatography to give compound 4 (525 mg, light yellow oil) in a yield of 53%.

MS m/z (ESI): 991.9 [M+1].

1H NMR (300MHz, CDCl3): δ 5.45 (t, 1H, J = 5.4 Hz), 4.53-4.40 (m, 1H), 4.20-3.95 (m, 4H), 3.01 (t, 4H, J=6.4 Hz), 2.57-2.36 (m, 6H), 2.11-1.78 (m, 9H), 1.76-0.85 (m, 86H), 0.70 (s, 3H).

### Example 5: Synthesis of Compound 5

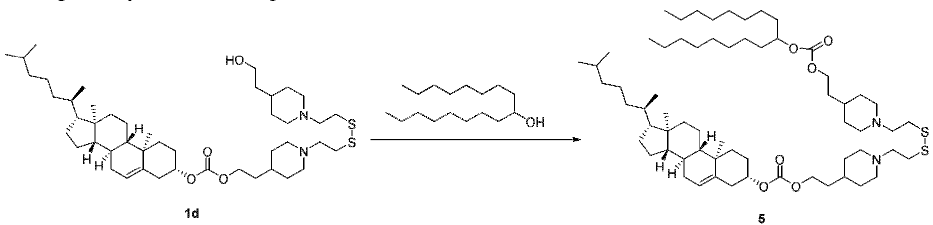

Compound 1d (5.0 g, 6 mmol) was dissolved in 30 mL of dichloromethane and 4-dimethylaminopyridine (1.3 g, 10 mmol) was added. Subsequently, phenyl *p*-nitrochlorofomiate (2.2 g, 11 mmol) was added in portions. The resulting mixture was stirred at room temperature for 3 h. 9-Hydroxyheptadecanol (2.5 g, 10 mmol) was added to the reaction solution, and the mixture was stirred at room temperature overnight. After the reaction was completed as detected by TLC, 20 mL of dichloromethane was added for dilution, and subsequently, the reaction solution was washed with 30 mL of saturated brine. The organic phase was dried over anhydrous sodium sulfate, filtered and concentrated to give product 5 (3.1 g, light yellow oil) in a yield of 48%.

MS m/z (ESI): 1071.8 [M+1].

1H NMR (300MHz, CDCl3): δ 5.45 (t, 1H, J = 5.4 Hz), 4.53-4.40 (m, 2H), 4.20-3.95 (m, 4H), 2.57-2.36 (m, 4H), 2.11-1.65 (m, 20H), 1.76-0.85 (m, 80H), 0.70 (s, 3H).

### Example 6: Synthesis of Compound 6

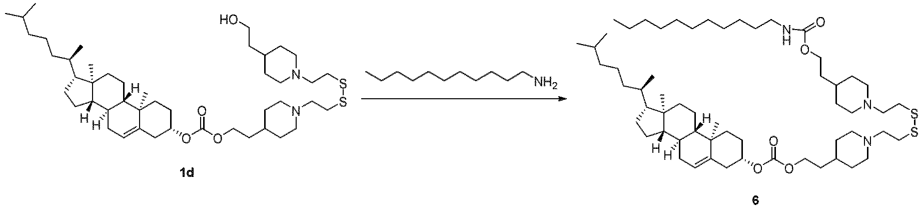

Compound 1d (5.0 g, 6 mmol) was dissolved in 30 mL of dichloromethane, and 4-dimethylaminopyridine (1.3 g, 10 mmol) was added. Subsequently, phenyl p-nitrochloroformate (2.2 g, 11 mmol) was added in portions. The resulting mixture was stirred at room temperature for 3 h. Undecylamine (1.7 g, 10 mmol) was added to the reaction solution, and the mixture was stirred at room temperature overnight. After the reaction was completed as detected by TLC, 20 mL of dichloromethane was added for dilution, and subsequently, the reaction solution was washed with 30 mL of saturated brine. The organic phase was dried over anhydrous sodium sulfate, filtered and concentrated to give product 6 (3.0 g, light yellow oil) in a yield of 51%.

MS m/z (ESI): 986.7 [M+1].

1H NMR (300MHz, CDCl3): δ 5.45 (t, 1H, J = 5.4 Hz), 4.53-4.40 (m, 1H), 4.20-3.95 (m, 4H), 3.19 (t, 2H, J = 5.4 Hz), 2.57-2.36 (m, 7H), 2.11-1.65 (m, 6H), 1.76-0.85 (m, 79H), 0.70 (s, 3H).

### Example 7: Synthesis of Compound 7

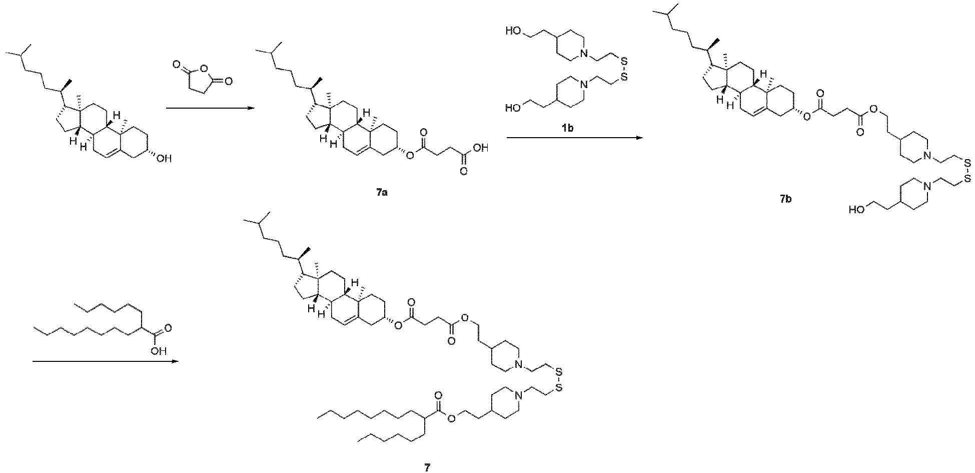

### Step 1: Synthesis of compound 7a

Cholesterol (2.0 g, 5 mmol) and succinic anhydride (1.0 g, 9 mmol) were dissolved in 20 mL of dichloromethane, and pyridine (1.6 g, 20.7 mmol) and DMAP (0.2 g, 2 mmol) were added. The reaction mixture was heated and refluxed for 6 h. Samples were taken for an HPLC assay to confirm the completion of the reaction. The reaction solution was concentrated under reduced pressure. 5% hydrochloric acid (10 mL) was added to the mixture, and the resulting mixture was extracted with diethyl ether (3 × 20 mL). The organic phases were combined, washed twice with brine, dried over anhydrous sodium sulfate, and concentrated. The organic phase was purified by column chromatography to give compound 7a (0.9 g, white solid) in a yield of 36%.

### Step 2: Synthesis of compound 7b

Compound 1b (0.75 g, 2 mmol) was dissolved in 20 mL of dichloromethane, and compound 7a (0.9 g, 2 mmol), EDC·HCL (0.9 g, 4 mmol), and DMAP (0.1 g, 0.8 mmol) were added. The resulting mixture was stirred at room temperature for 16-20 h, and the reaction was completed as detected by CAD. The reaction solution was concentrated, dissolved by the addition of n-hexane, and washed twice with acetonitrile. The n-hexane phase was concentrated to dryness and separated by column chromatography to give compound 7b (0.68 g, light yellow oil) in a yield of 40%.

### Step 3: Synthesis of compound 7

Compound 7b (600 mg, 0.7 mmol) was dissolved in 10 mL of dichloromethane, and 2-hexyldecanoic acid (180 mg, 0.7 mmol), EDC·HCL (270 mg, 1.4 mmol), and DMAP (33 mg, 0.3 mmol) were added. The resulting mixture was stirred at room temperature for 16-20 h, and the reaction was completed as detected by CAD. The reaction solution was concentrated, dissolved by the addition of n-hexane, and washed twice with acetonitrile. The n-hexane phase was concentrated to dryness and separated by column chromatography to give compound 7 (364 mg, light yellow oil) in a yield of 48%.

MS m/z (ESI): 1083.8 [M+1].

1H NMR (300MHz, CDCl3): δ 5.45 (t, 1H, J = 5.4 Hz), 4.53-4.40 (m, 1H), 4.20-3.95 (m, 4H), 2.95-2.80 (m, 8H), 2.71-2.50 (m, 14H), 2.47-2.36 (m, 5H), 2.11-1.78 (m, 6H), 1.76-0.85 (m, 72H), 0.70 (s, 3H).

### Example 8: Synthesis of Compound 8

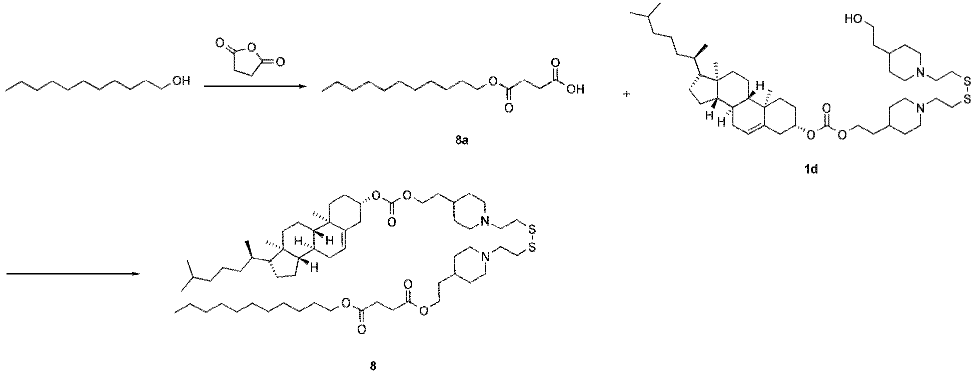

### Step 1: Synthesis of compound 8a

Undecanol (0.3 g, 5 mmol) and succinic anhydride (1.0 g, 9 mmol) were dissolved in 10 mL of dichloromethane, and pyridine (1.6 g, 20.7 mmol) and DMAP (0.2 g, 2 mmol) were added. The reaction mixture was heated and refluxed for 6 h. Samples were taken for an HPLC assay to confirm the completion of the reaction. The reaction system was concentrated under reduced pressure. 5% hydrochloric acid (10 mL) was added to the mixture, and the resulting mixture was extracted with diethyl ether (3 × 20 mL). The organic phases were combined, washed twice with brine, dried over anhydrous sodium sulfate, concentrated, and purified by column chromatography to give compound 8a (0.6 g, light yellow oil) in a yield of 43%.

### Step 2: Synthesis of compound 8

Compound 8a (600 mg, 2.2 mmol) was dissolved in 10 mL of dichloromethane, and compound 1d (1.7 g, 2.2 mmol), EDC·HCL (845 mg, 4.4 mmol), and DMAP (100 mg, 0.9 mmol) were added. The resulting mixture was stirred at room temperature for 16-20 h, and the reaction was completed as detected by CAD. The reaction solution was concentrated, dissolved by the addition of n-hexane, and washed twice with acetonitrile. The n-hexane phase was concentrated to dryness and separated by column chromatography to give compound 8 (1.0 g, light yellow oil) in a yield of 45%.

MS m/z (ESI): 1043.7 [M+1].

1H NMR (300MHz, CDCl3): δ 5.45 (t, 1H, J = 5.4 Hz), 4.53-4.40 (m, 1H), 4.20-3.95 (m, 6H), 2.73-2.66 (m, 8H), 2.71-2.50 (m, 14H), 2.47-2.36 (m, 4H), 2.11-1.78 (m, 6H), 1.76-0.85 (m, 63H), 0.70 (s, 3H).

### Example 9: Synthesis of Compound 9

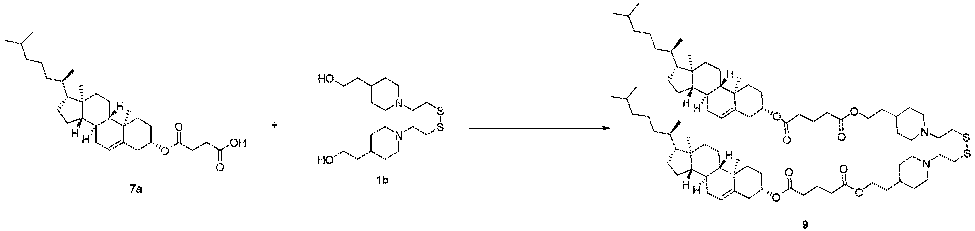

Compound 1b (0.75 g, 2 mmol) was dissolved in 20 mL of dichloromethane, and compound 7a (1.8 g, 4 mmol), EDC·HCL (1.8 g, 8 mmol), and DMAP (0.2 g, 1.6 mmol) were added. The resulting mixture was stirred at room temperature for 16-20 h, and the reaction was completed as detected by CAD. The reaction solution was concentrated until completion, dissolved by the addition of n-hexane, and washed twice with acetonitrile. The *n-*hexane phase was concentrated to dryness and purified by column chromatography to give compound 9 (1.2 g, light yellow oil) in a yield of 45%.

MS m/z (ESI): 1342.0 [M+1].

1H NMR (300MHz, CDCl3): δ 5.45 (t, 2H, J = 5.4 Hz), 4.53-4.40 (m, 2H), 4.20-3.95 (m, 4H), 2.73-2.66 (m, 4H), 2.71-2.36 (m, 4H), 2.11-1.78 (m, 12H), 1.76-0.85 (m, 102H), 0.70 (s, 6H).

### Example 10: Preparation and Characterization of mRNA-LNPs

The lipid compound of the present invention, the neutral lipid (phospholipid), and the PEG lipid were dissolved in an ethanol solution at a certain molar ratio (see Table 1 for the specific formulation). Commercial SM-102, compound B-2 disclosed in patent document CN103930398B, compound ss-OP disclosed in patent document CN114929205A, and lipid compound 39 disclosed in patent document CN114787127A were used in the comparative examples. The mRNA was dissolved in 25 mM acetic acid-sodium acetate buffer solution at pH 4 to a make a final concentration of 135 ng/µL. The mRNA-LNP complexes were prepared using a microfluidic nano-preparation system with the flow rate ratio of the ethanol phase (lipid mixture) to the aqueous phase (mRNA) being 1:3, and subsequently, the mRNA-LNP complexes were diluted with 10× sodium acetate buffer solution at pH 7.5, ultrafiltered, concentrated, and sterilely filtered to give experimental samples. Samples were taken and assayed for the mRNA encapsulation efficiency using a Quanti^{™}-Ribogreen method. The average particle size, PDI, and Zeta potential were assayed using a dynamic light scattering nanoparticle size analyzer.

**Table 1: Three-component LNP lipid formulations based on cholesterol cationic lipid compounds**

| No. | Lipid formulation (molar percent) | Nitrogen-to-phosphorus ratio |
|---|---|---|
| LNP1 | Compound 1:DSPC:DMG-PEG2000 = 49.25:49.25:1.5 | 8 |
| LNP2 | Compound 2:DSPC:DMG-PEG2000 = 49.25:49.25:1.5 | 8 |
| LNP3 | Compound 3:DSPC:DMG-PEG2000 = 49.25:49.25:1.5 | 8 |
| LNP4 | Compound 4:DSPC:DMG-PEG2000 = 49.25:49.25:1.5 | 8 |
| LNP5 | Compound 5:DSPC:DMG-PEG2000 = 49.25:49.25:1.5 | 8 |
| LNP6 | Compound 6:DSPC:DMG-PEG2000 = 49.25:49.25:1.5 | 8 |
| LNP7 | Compound 7:DSPC:DMG-PEG2000 = 49.25:49.25:1.5 | 8 |
| LNP8 | Compound 8:DSPC:DMG-PEG2000 = 49.25:49.25:1.5 | 8 |
| LNP9 | Compound 9:DSPC:DMG-PEG2000 = 49.25:49.25:1.5 | 8 |
| LNP10 | SM-102:DSPC:DMG-PEG2000=49.25:49.25:1.5 | 8 |
| LNP11 | Compound B-2:DSPC:DMG-PEG2000 = 49.25:49.25:1.5 | 8 |
| LNP12 | Compound ss-OP:DSPC:DMG-PEG2000 = 49.25:49.25:1.5 | 8 |
| LNP13 | Compound 39:DSPC:DMG-PEG2000 = 49.25:49.25:1.5 | 8 |

The compounds used in the comparative examples had the following structures, and all the comparative compounds were synthesized by Tianjin Jenkem Technology Co., Ltd. with reference to the related patent documents. The structural characterization demonstrated that the compounds were synthesized correctly.

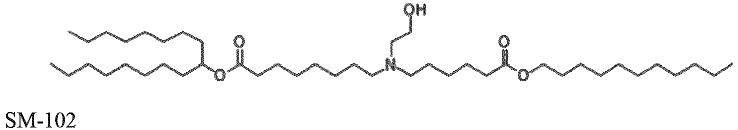

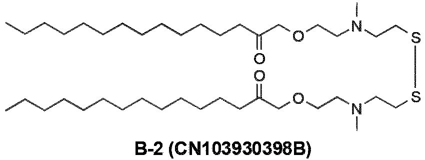

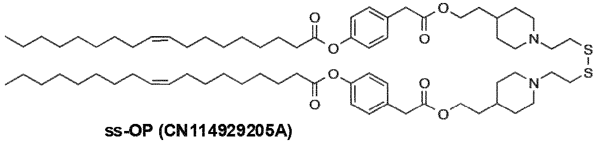

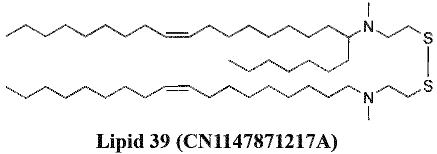

**Example 11:** Preparation, Characterization and *In Vitro* Cell Transfection Activity Evaluation of Luc-mRNA-LNPs We constructed Luc-mRNA-LNPs using luciferase gene Luc-mRNA with reference to the preparation method provided in Example 10 and the lipid formulations provided in Table 1. The data on the encapsulation efficiencies, average particle sizes, PDIs, and zeta potentials of the Luc-mRNA-LNPs are shown in Table 2. The results demonstrated that the three-component LNPs constructed by the cationic lipid compound provided by the present invention, the phospholipid, and the PEG lipid were capable of effectively encapsulating mRNA. The particle sizes of the LNPs were 80-120 nm; the PDIs of the LNPs were all less than 0.2; the mRNA encapsulation efficiencies of the LNPs were all higher than 80%. However, based on the LNP formulations provided by the present invention, commercial lipid SM-102 and other disclosed compounds were all incapable of effectively encapsulating mRNA. The encapsulation efficiencies of these controls were all lower than 20%.

**Table 2: Physicochemical parameters of the Luc-mRNA-LNPs**

| No. | Average particle size (nm) | PDI | Zeta potential (mV) | Encapsulation efficiency (%) |
|---|---|---|---|---|
| LNP1 | 105.2 | 0.1247 | -3.251 | 87.11 |
| LNP2 | 122.1 | 0.1677 | -3.5845 | 83.98 |
| LNP3 | 114.9 | 0.07915 | 1.3469 | 93.48 |
| LNP4 | 100.2 | 0.1611 | 4.091 | 89.40 |
| LNP5 | 89.15 | 0.1280 | 8.343 | 87.70 |
| LNP6 | 116.4 | 0.06409 | -5.0535 | 92.76 |
| LNP7 | 102.6 | 0.1161 | -3.4055 | 91.60 |
| LNP8 | 91.91 | 0.04902 | -4.252 | 90.85 |
| LNP9 | 115.42 | 0.1491 | -3.986 | 82.29 |
| LNP10 | 82.33 | 0.1193 | -3.5855 | 0.89 |
| LNP 11 | 96.54 | 0.1223 | -3.611 | 5.00 |
| LNP12 | 136.3 | 0.1087 | -9.297 | 16.36 |
| LNP 13 | 101.9 | 0.1033 | -10.485 | 7.38 |

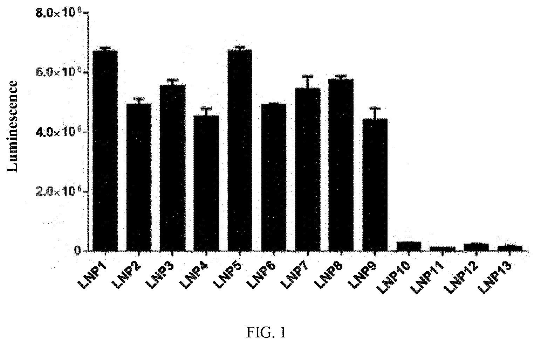

HEK293T cells at a concentration of 6.5 × 10⁵ cells/mL were seeded into a 96-well cell culture plate at 100 µL/well. 24 hours later, the cells in each well were transfected with 100 ng of Luc-mRNA-LNP, and subsequently, the cell culture plate was placed in a cell incubator with 5% CO₂ at 37 °C for culture. The cells in the negative control group were transfected with an equal volume of normal saline. After the completion of the culture, a bioluminescence assay was performed with reference to the instruction manual of Fire-Lumi^{™} luciferase detection kit. The results are shown in FIG. 1. The results demonstrated that the three-component LNPs constructed using the cationic lipid compounds of the present invention had better cell transfection efficiency, and were capable of achieving high expression of Luc-mRNA in cells, with an expression level significantly superior to that of the control group.

### Example 12: mRNA-LNP Animal Immunization Assay

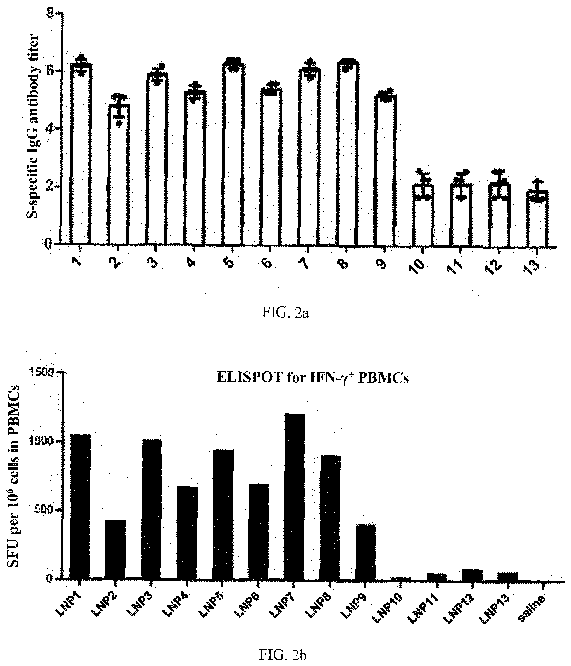

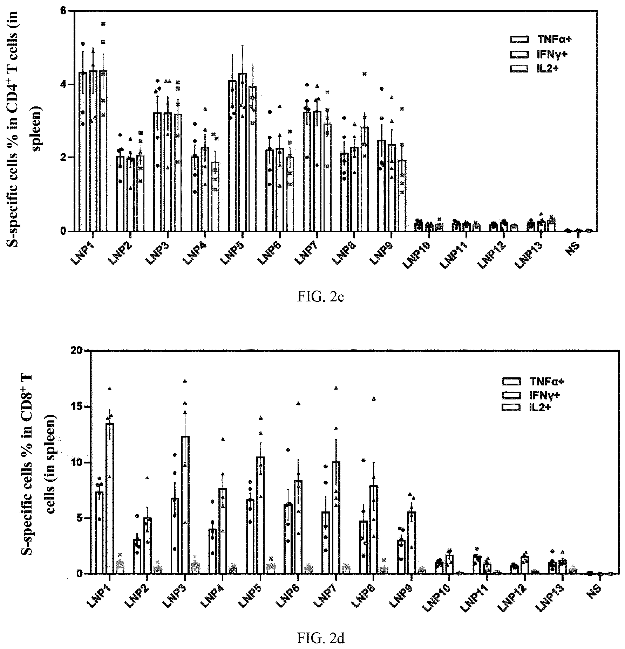

We evaluated the immunological activity of the mRNA-LNPs in mice using the novel coronavirus S protein mRNA. The used LNP formulations are as shown in Table 1. The novel coronavirus antigen S protein mRNA-LNPs were prepared according to the preparation method provided in Example 10. Female BALB/c mice aged 6-8 weeks were grouped at 5 mice/group randomly, and immunized by means of intramuscular injection of hind leg. The immunization was performed on day 0 and day 14 at an immunization dose of 5 µg of mRNA-LNPs per mouse. Blood was collected on day 28 of immunization and serum was isolated. The mice were sacrificed to take the spleen tissues. The titers of the specific IgG antibodies targeting the novel coronavirus S protein antigen encoded by mRNA were assayed using an ELISA method. PBMCs were isolated and pooled for an IFN-γ+ cell ELISPOT assay, and the spleen tissues were subjected to an ICS assay (IL-2, TNF-α, and IFN-γ) for antigen-specific CD4+ T and CD8+ T cells. The results are as shown in FIGs. 2a, 2b, 2c, and 2d. The LNP delivery system constructed using the cationic lipid compounds provided by the present invention is capable of inducing a higher antigen-specific antibody titer and stronger cellular immunity when delivering the novel coronavirus antigen mRNA to mice, and has a significantly better effect compared to the prior art.

Finally, it should be noted that, the above examples are only used to illustrate the technical schemes of the present invention, but not to limit the present invention. Although the present invention is described in detail with reference to the examples described above, it will be understood by those skilled in the art that, the technical solutions in the examples described above can still be modified, or some or all of the technical features can be equivalently replaced; and these modifications or replacements do not make the technical solutions corresponding thereto depart from the scope of the technical solutions in the examples of the present invention.

## Claims

1. A lipid compound as shown in formula (I), 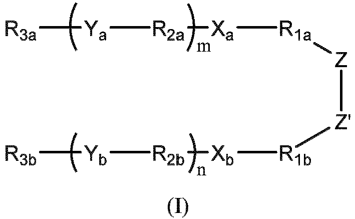 or a stereoisomer, a tautomer, and a pharmaceutically acceptable salt thereof, wherein,
Xₐ and X_{b} are each independently selected from 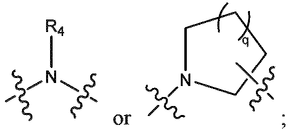
R₄ is C₁-C₆ alkyl; q is an integer of 1 or 2;
Z and Z' are each independently selected from S, C, or a chemical bond;
R₁ₐ and R_{1b} are each independently selected from C₁₋₆ alkylene or C₃-C₈ cycloalkylene;
R₂ₐ and R_{2b} are each independently selected from C₁₋₆ alkylene;
Yₐ and Y_{b} are independently selected from: -O-, -O(C=O)O-, -(C=O)NRa-, -NRa(C=O)-, -NRa-, -O(C=O)-, - (C=O)O-, -C(=O)-, -S(O)x-, -S-S-, -C(=O)S-, -SC(=O)-, -NRaC(=O)NRa-, -OC(=O)NRa-, -NRaC(=O)O-, - OC(=O)S-, or -SC(=O)O-;
Rₐ is H or C₁-C₁₂ alkyl;
m and n are each independently selected from 1, 2, or 3;
R₃ₐ and R_{3b} are selected from a naturally occurring or non-naturally occurring steroid group, a fat-soluble vitamin residue, C₆-C₂₄ alkyl, C₆-C₂₄ monoalkenyl or polyalkenyl, or C₆-C₂₄ monoalkynyl or polyalkynyl, wherein CH₂ on the alkyl, alkenyl, or alkynyl is optionally replaced by one or more oxygen atoms;
provided that at least one of R₃ₐ and R_{3b} is a naturally occurring or non-naturally occurring steroid group.

2. The lipid compound according to claim 1, wherein the lipid compound is a compound as shown in formula (II), 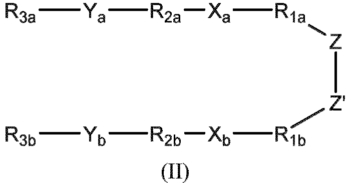 or a stereoisomer, a tautomer, and a pharmaceutically acceptable salt thereof, wherein,
Xₐ and X_{b} are each independently selected from 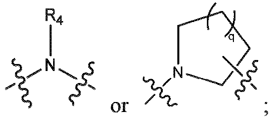
R₄ is C₁-C₆ alkyl; q is an integer of 1 or 2;
Z and Z' are each independently selected from S, C, or a chemical bond;
R₁ₐ and R_{1b} are each independently selected from C₁₋₆ alkylene or C₃-C₈ cycloalkylene;
R₂ₐ and R_{2b} are each independently selected from C₁₋₆ alkylene;
Yₐ and Y_{b} are independently selected from: -O-, -O(C=O)O-, -(C=O)NRa-, -NRa(C=O)-, -NRa-, -O(C=O)-, - (C=O)O-, -C(=O)-, -S(O)x-, -S-S-, -C(=O)S-, -SC(=O)-, -NRaC(=O)NRa-, -OC(=O)NRa-, -NRaC(=O)O-, - OC(=O)S-, or -SC(=O)O-;
Rₐ is H or C₁-C₁₂ alkyl;
R₃ₐ and R_{3b} are selected from a naturally occurring or non-naturally occurring steroid group, a fat-soluble vitamin residue, C₆-C₂₄ alkyl, C₆-C₂₄ monoalkenyl or polyalkenyl, or C₆-C₂₄ monoalkynyl or polyalkynyl, wherein CH₂ on the alkyl, alkenyl, or alkynyl is optionally replaced by one or more oxygen atoms;
provided that at least one of R₃ₐ and R_{3b} is a naturally occurring or non-naturally occurring steroid group.

3. The lipid compound according to claim 1 or 2, or a stereoisomer, a tautomer, and a pharmaceutically acceptable salt thereof, wherein, Xₐ and X_{b} are selected from 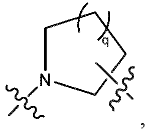 wherein q is an integer of 1 or 2.

4. The lipid compound according to any one of the preceding claims, or a stereoisomer, a tautomer, and a pharmaceutically acceptable salt thereof, wherein,
Z and Z' are each independently selected from S or C.

5. The lipid compound according to any one of the preceding claims, wherein the lipid compound is a compound as shown in formula (III-1), formula (III-2), or formula(III-3), 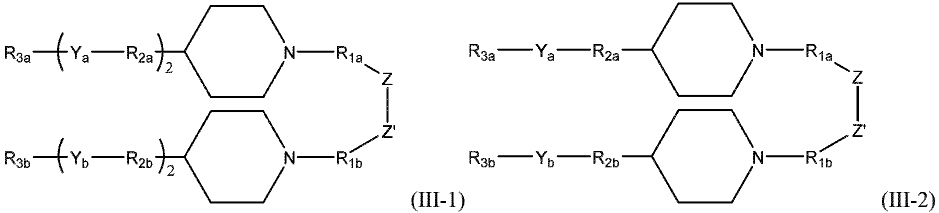 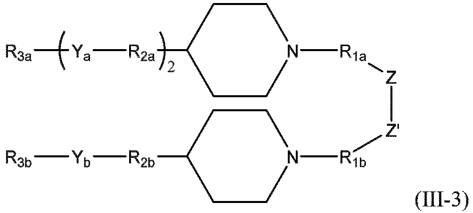 or a stereoisomer, a tautomer, and a pharmaceutically acceptable salt thereof, wherein Z, Z', Yₐ, Y_{b}, R₁ₐ, R_{1b}, R₂ₐ, R_{2b}, R₃ₐ, and R_{3b} are as defined in the preceding claims.

6. The lipid compound according to any one of the preceding claims, or a stereoisomer, a tautomer, and a pharmaceutically acceptable salt thereof; wherein,
R₁ₐ and R_{1b} are each independently C₁-C₆ alkylene;
R₂ₐ and R_{2b} are each independently C₁-C₃ alkylene;
Yₐ and Y_{b} are each independently selected from: -O-, -O(C=O)O-, -O(C=O)-, -(C=O)O-, or -C(=O)-;
R₃ₐ and R_{3b} are selected from a naturally occurring or non-naturally occurring steroid group, a fat-soluble vitamin residue, C₆-C₂₄ alkyl, C₆-C₂₄ monoalkenyl or polyalkenyl, or C₆-C₂₄ monoalkynyl or polyalkynyl, wherein CH₂ on the alkyl, alkenyl, or alkynyl is optionally replaced by one or more oxygen atoms,
provided that at least one of R₃ₐ and R_{3b} is a naturally occurring or non-naturally occurring steroid group.

7. The lipid compound according to any one of the preceding claims, wherein the steroid is selected from: avenasterol, β-sitosterol, brassicasterol, ergocalciferol, campesterol, cholestanol, cholesterol, an oxidized form of cholesterol, a reduced form of cholesterol, coprostanol, dehydrocholesterol, desmosterol, dihydroergocalciferol, dihydrocholesterol, dihydroergosterol, dinosterol, epicholesterol, ergosterol, fucosterol, hexahydrolumisterol, hydroxycholesterol, lanosterol, lumisterol, saringosterol, sitostanol, sitosterol, stigmastanol, stigmasterol, cholic acid, glycocholic acid, taurocholic acid, deoxycholic acid, lithocholic acid, and/or an alkyl ester of lithocholic acid.

8. The lipid compound according to any one of the preceding claims, wherein the steroid group is selected from: 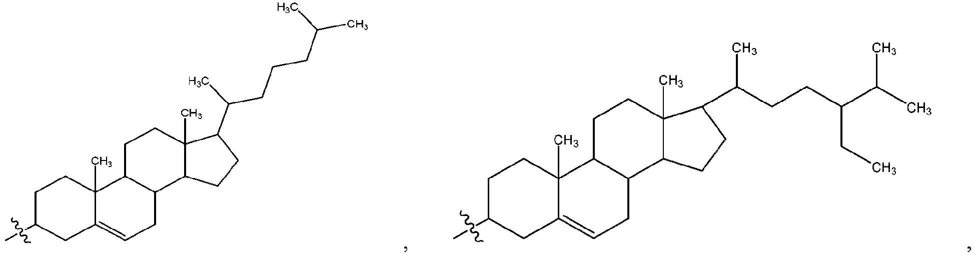 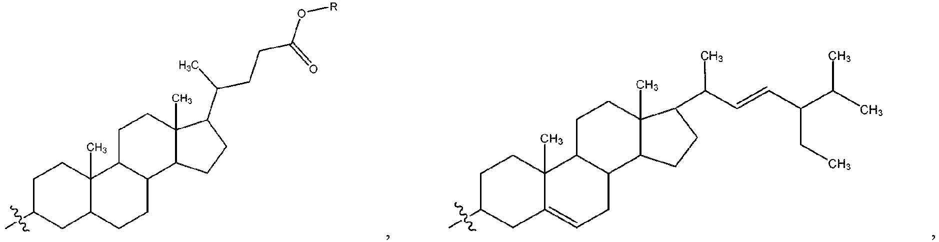 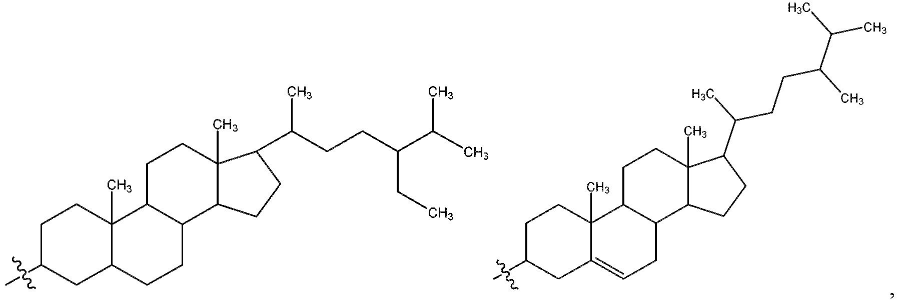 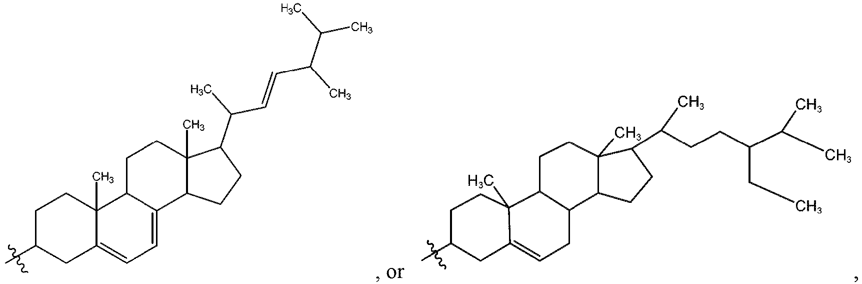 wherein R is C₁₋₂₀ alkyl.

9. The lipid compound according to any one of the preceding claims, wherein the lipid compound is selected from: 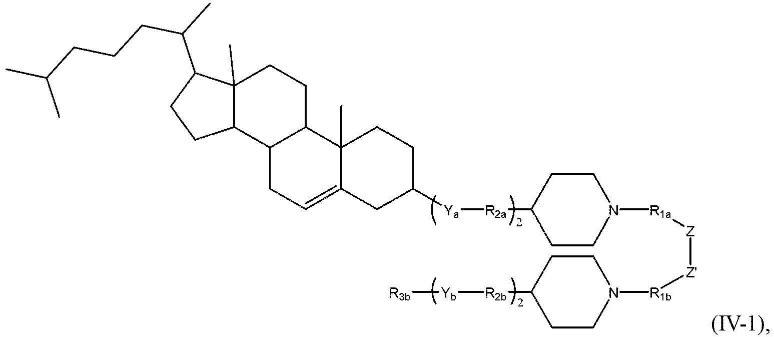 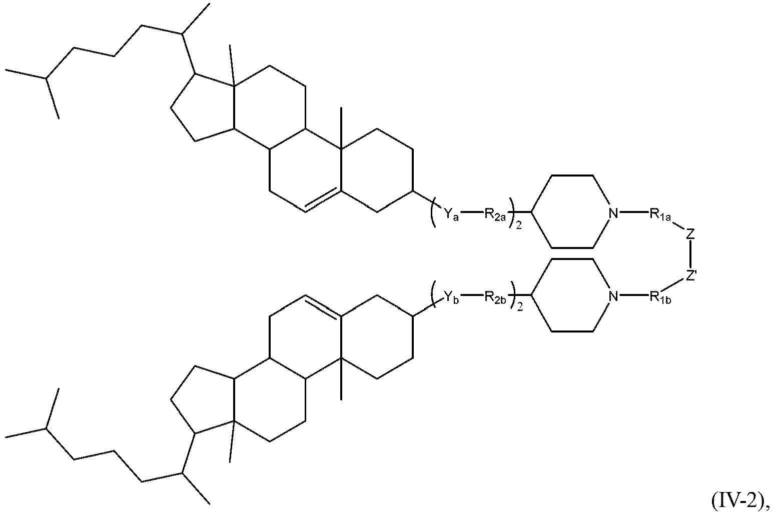 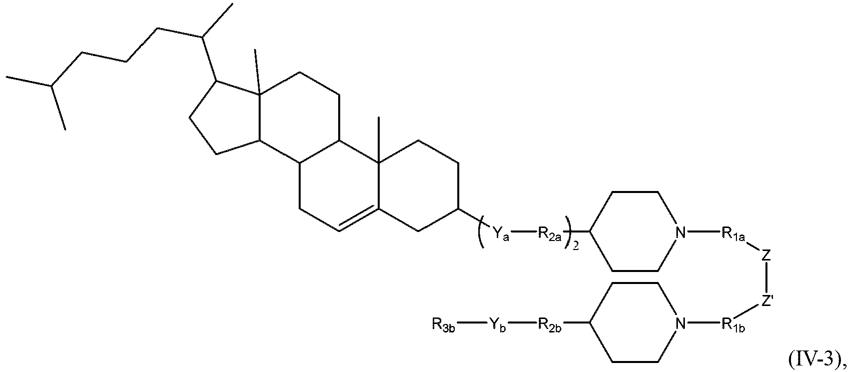 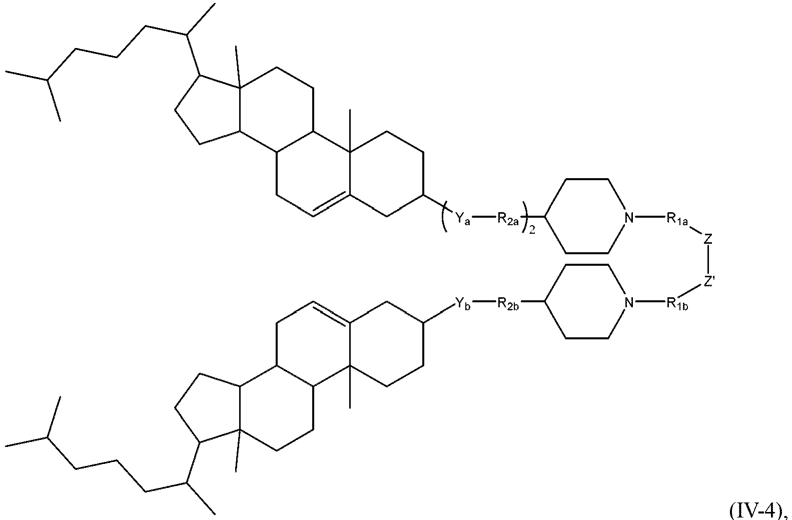 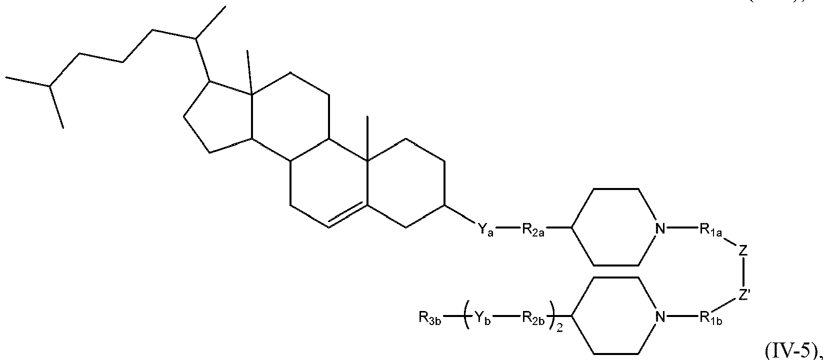 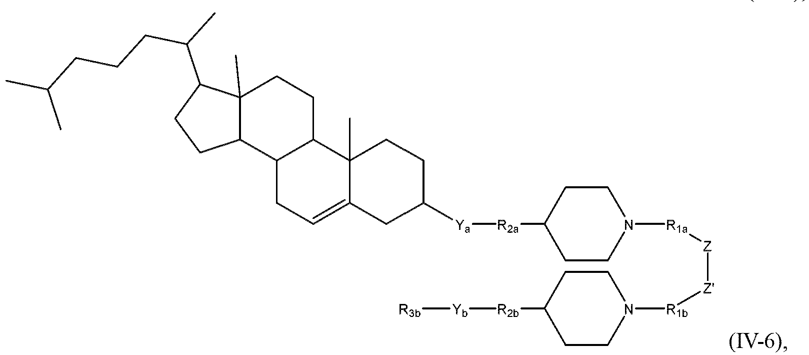 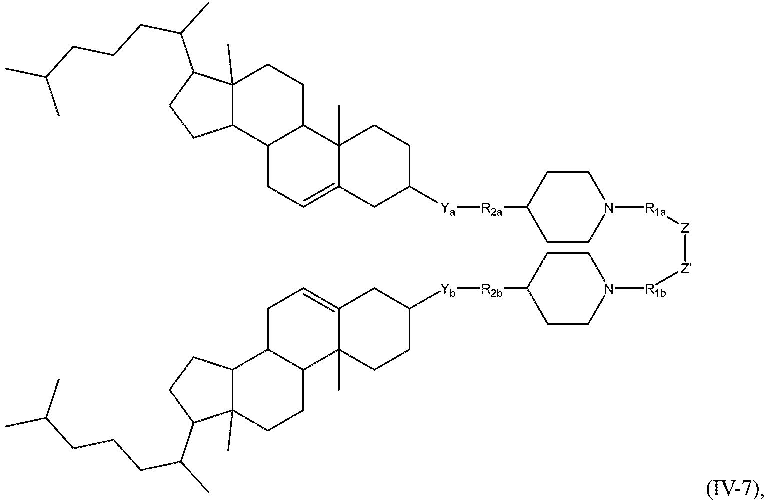 or a stereoisomer, a tautomer, and a pharmaceutically acceptable salt thereof, wherein R_{3b} is selected from a fat-soluble vitamin residue, C₆-C₂₄ alkyl, C₆-C₂₄ monoalkenyl or polyalkenyl, or C₆-C₂₄ monoalkynyl or polyalkynyl, wherein CH₂ on the alkyl, alkenyl, or alkynyl is optionally replaced by one or more oxygen atoms; Z, Z', Yₐ, Y_{b}, R₁ₐ, R_{1b}, R₂ₐ, and R_{2b} are as defined in the preceding claims.

10. The lipid compound according to any one of the preceding claims, wherein the lipid compound is selected from: 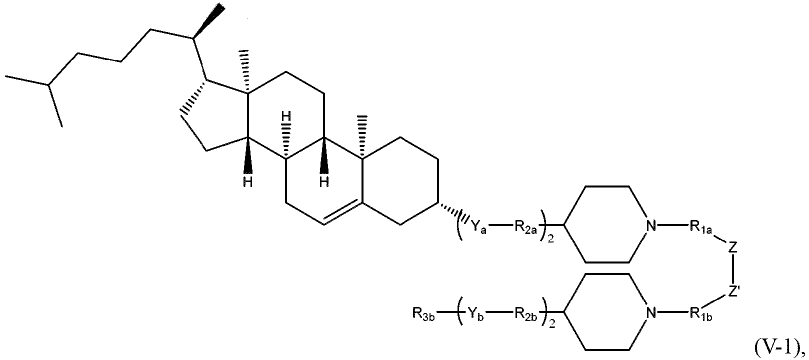 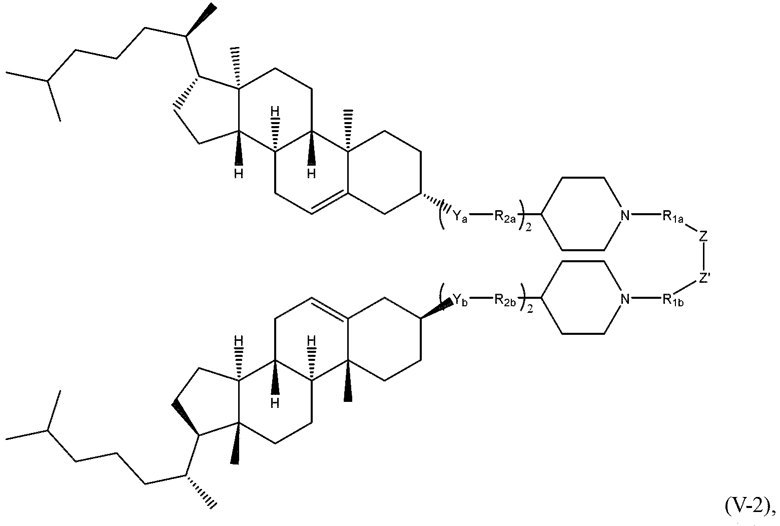 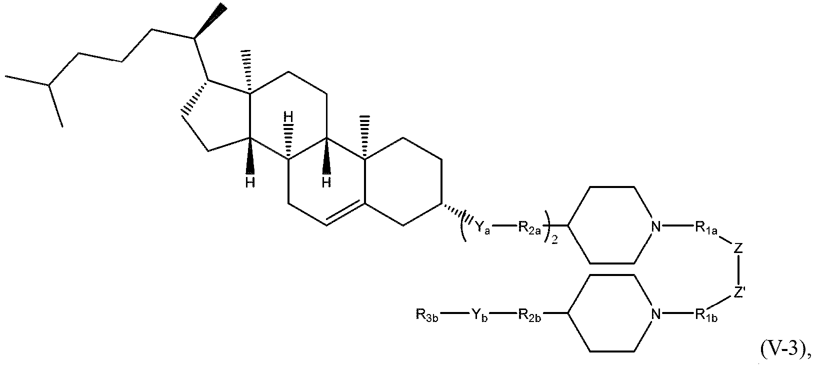 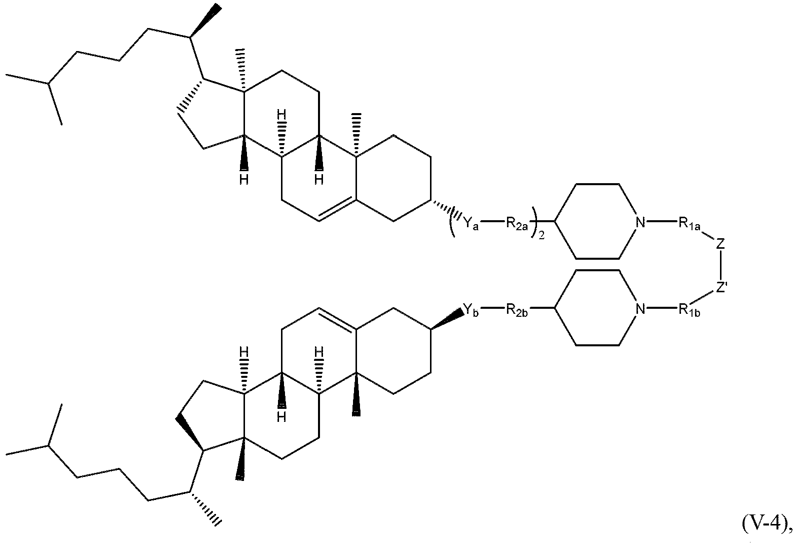 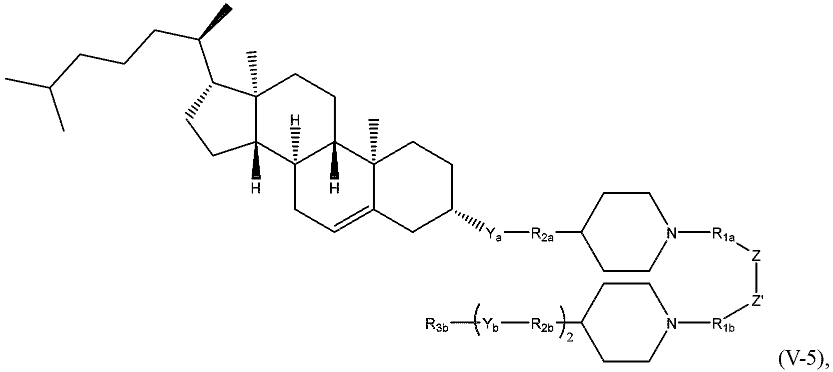 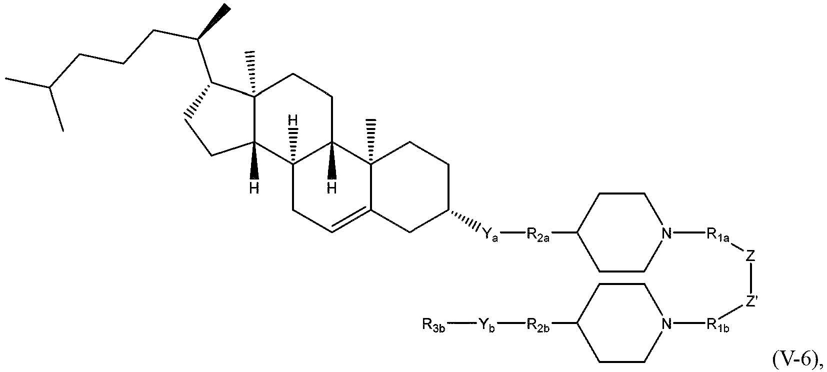 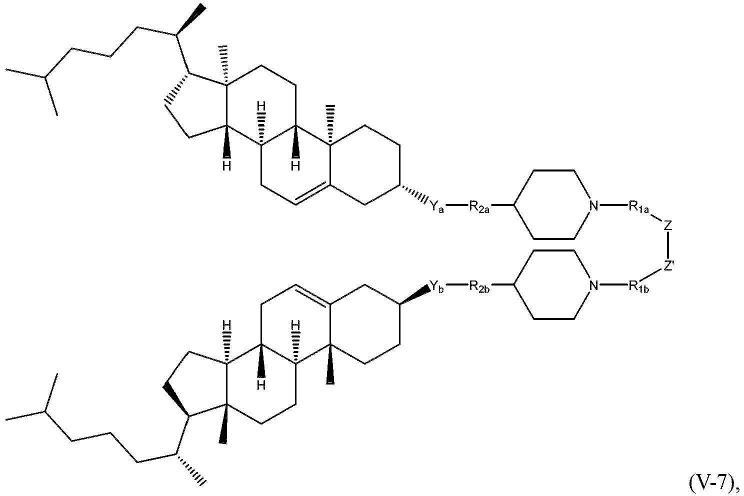 or a stereoisomer, a tautomer, and a pharmaceutically acceptable salt thereof, wherein R_{3b} is selected from a fat-soluble vitamin residue, C₆-C₂₄ alkyl, C₆-C₂₄ monoalkenyl or polyalkenyl, or C₆-C₂₄ monoalkynyl or polyalkynyl, wherein CH₂ on the alkyl, alkenyl, or alkynyl is optionally replaced by one or more oxygen atoms; Z, Z', Yₐ, Y_{b}, R₁ₐ, R_{1b}, R₂ₐ, and R_{2b} are as defined in the preceding claims.

11. The lipid compound according to any one of the preceding claims, or a stereoisomer, a tautomer, and a pharmaceutically acceptable salt thereof, wherein R_{3b} is selected from: , 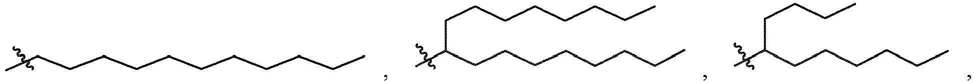 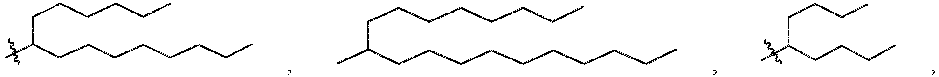 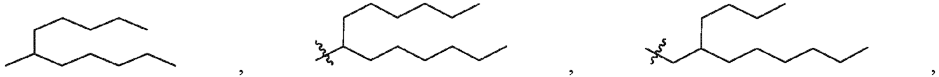 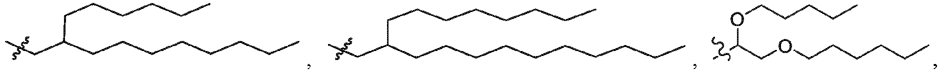 or

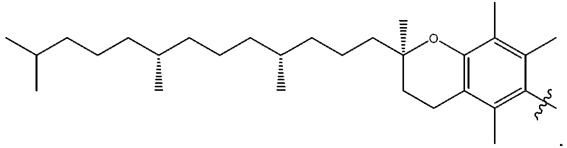

12. The lipid compound according to claim 1, wherein the lipid compound is selected from: 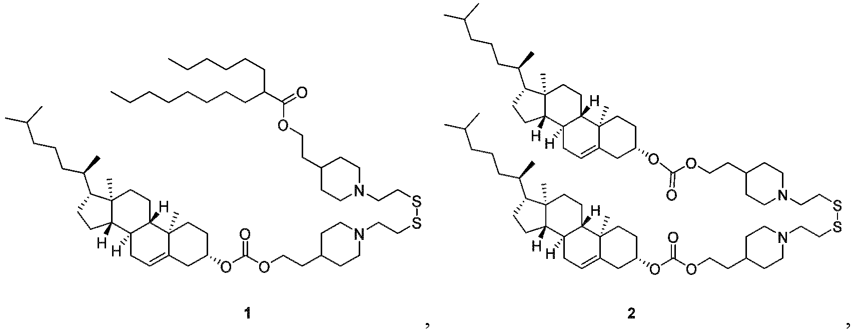 or 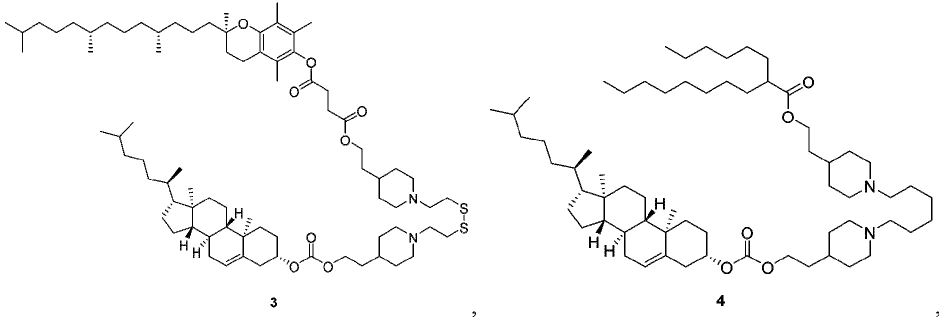 or 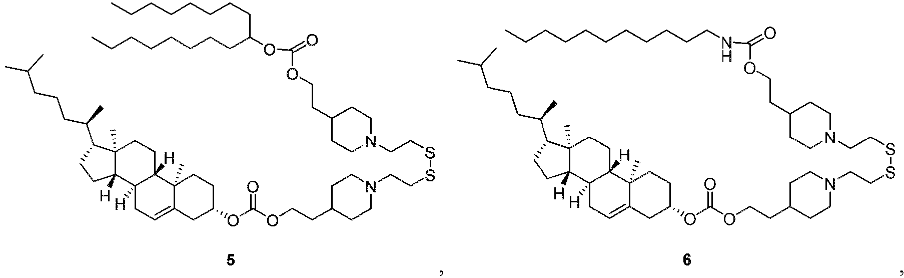 or 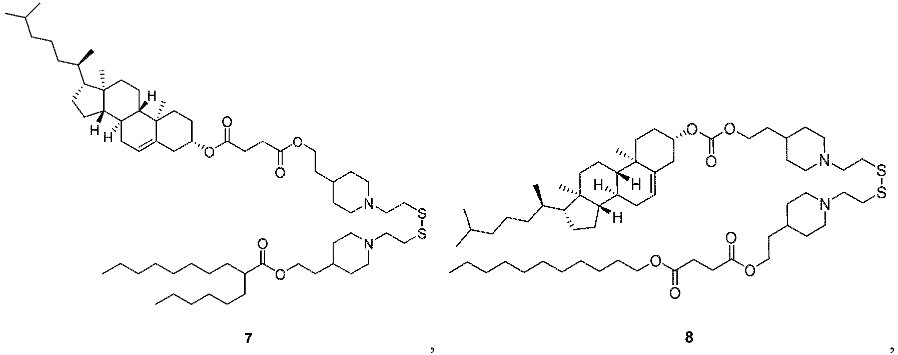 or 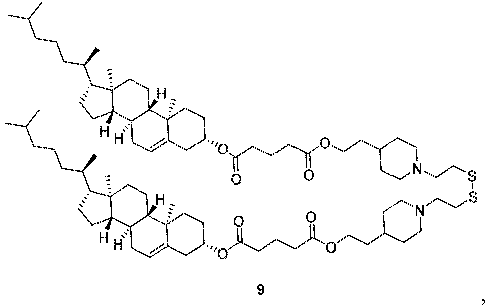 or a stereoisomer, a tautomer, and a pharmaceutically acceptable salt thereof.

13. A lipid nanoparticle (LNP), comprising the lipid compound according to any one of the preceding claims.

14. The LNP according to claim 13, further comprising a polyethylene glycol lipid and at least one second lipid, wherein the second lipid is selected from: a neutral lipid, a zwitterionic lipid, and/or an anionic lipid.

15. The LNP according to claim 14, wherein the polyethylene glycol lipid is selected from: 2-[(polyethylene glycol)-2000]-N,N-ditetradecylacetamide, 1,2-dimyristoyl-sn-glycerol methoxypolyethylene glycol, 1,2-distearoyl-sn-glycero-3-phosphoethanolamine-N-[amino(polyethylene glycol)], PEG-disterol glycerol, PEG-dipalmitoyl, PEG-dioleyl, PEG-distearyl, PEG-diacylglycerol amide, PEG-dipalmitoyl phosphatidylethanolamine (PEG-DPPE), or PEG-1,2-dimyristoyloxypropyl-3-amine;
the neutral lipid is selected from 1,2-distearoyl-sn-glycero-3-phosphocholine, 1,2-dipalmitoyl-sn-glycero-3-phosphocholine, 1,2-dioleoyl-sn-glycerol-3-phosphoethanolamine, 1,2-dipalmitoyl-sn-glycero-3-phosphoethanolamine, 1,2-dimyristoyl-sn-glycero-3-phosphoethanolamine, 2-dioleoyl-sn-glycero-3-phospho-(1'-rac-glycerol), oleoyl phosphatidylcholine, or 1-palmitoyl-2-oleoyl phosphatidylethanolamine.

16. The LNP according to claim 15, wherein a molar ratio of the lipid according to any one of the preceding claims to the second lipid to the polyethylene glycol lipid is in the range of 10:20:1 to 60:80:30; and/or a nitrogen-to-phosphorus ratio of the LNP is in the range of 1:1 to 15:1.

17. The LNP according to any one of the preceding claims, having a diameter of 15-300 nm.

18. Use of the lipid compound according to any one of claims 1-12 and/or the LNP according to any one of claims 13-17 in the preparation of a bioactive substance delivery system.

19. The use according to claim 18, wherein the bioactive substance is a DNA or an RNA, the RNA is selected from an antisense RNA, an saRNA, an mRNA, an lncRNA, an miRNA, an siRNA, a piRNA, a gRNA, a tsRNA, a circRNA, and a self-replicating mRNA.

20. The use according to claim 19, wherein the bioactive substance delivery system is an mRNA vaccine used for preventing cancer, viral infection, bacterial infection, and fungal infection; preferably, the virus is selected from: norovirus, Ebola virus, coronavirus, cytomegalovirus, Dengue virus, Zika virus, coxsackievirus, enterovirus, hepatitis virus, herpes simplex virus, human papilloma virus, influenza virus, Marburg virus, measles virus, poliovirus, rabies virus, rotavirus, or measles virus.

21. A medicament, comprising the bioactive substance according to claim 17 and the LNP according to any one of claims 11-15, and a pharmaceutically acceptable auxiliary material.

22. The medicament according to claim 21, wherein the medicament is a gene drug.
